(19) Europäisches Patentamt / European Patent Office / Office européen des brevets

(11) **EP 3 792 362 B1**

(12) **EUROPEAN PATENT SPECIFICATION**

(45) Date of publication and mention of the grant of the patent: **30.09.2026 Bulletin 2026/40**

(21) Application number: **18902067.0**

(22) Date of filing: **23.01.2018**

(51) International Patent Classification (IPC): ***C12Q 1/68*** *(2018.01)*

(52) Cooperative Patent Classification (CPC): **C12Q 1/6886;** C12Q 2600/112; C12Q 2600/118; C12Q 2600/154

(86) International application number: **PCT/CN2018/073821**

(87) International publication number: **WO 2019/144277 (01.08.2019 Gazette 2019/31)**

(54) **METHOD AND KIT FOR IDENTIFYING STATE OF COLORECTAL CANCER**

VERFAHREN UND KIT ZUR IDENTIFIZIERUNG DES ZUSTANDS VON KOLOREKTALKREBS

MÉTHODE ET KIT POUR IDENTIFIER L'ÉTAT D'UN CANCER COLORECTAL

(84) Designated Contracting States: **AL AT BE BG CH CY CZ DE DK EE ES FI FR GB GR HR HU IE IS IT LI LT LU LV MC MK MT NL NO PL PT RO RS SE SI SK SM TR**

(43) Date of publication of application: **17.03.2021 Bulletin 2021/11**


(73) Proprietor: **Excellen Medical Technology Co., Ltd. Science and Technology Park, Changpin District Beijing 102200 (CN)**

(72) Inventors:
- **LI, Mingming Beijing 102200 (CN)**
- **LI, Shuyu Beijing 102200 (CN)**
- **CHEN, Yanli Beijing 102200 (CN)**
- **XU, Chunye Beijing 102200 (CN)**
- **PU, Jue Beijing 102200 (CN)**

(74) Representative: **Glück Kritzenberger Patentanwälte PartGmbB Franz-Mayer-Str. 16a 93053 Regensburg (DE)**



(56) References cited:
**WO-A1-2018/087129 WO-A2-2011/126768 WO-A2-2011/126768 RU-C1- 2 630 669 RU-C1- 2 630 669**

- **RASMUSSEN SIMON LADEFOGED ET AL: "Hypermethylated DNA, a circulating biomarker for colorectal cancer detection", vol. 12, no. 7, 10 July 2017 (2017-07-10), pages e0180809, XP055792289, Retrieved from the Internet <URL:https://storage.googleapis.com/plos-corpus-prod/10.1371/journal.pone.0180809/1/pone.0180809.pdf?X-Goog-Algorithm=GOOG4-RSA-SHA256&X-Goog-Credential=wombat-sa@plos-prod.iam.gserviceaccount.com/20210401/auto/storage/goog4_request&X-Goog-Date=20210401T115148Z&X-Goog-Expires=3600&X-Goog-SignedHeaders=ho> DOI: 10.1371/journal.pone.0180809**


Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).




Processed by Luminess, 75001 PARIS (FR)

## Description

FIELD OF THE INVENTION

The present invention relates to a method and a kit for identifying a colorectal cancer status in a subject.

BACKGROUND

Colorectal cancer is one of the most common diseases today. About 1.2 million patients worldwide are diagnosed with colorectal cancer each year, and more than 600,000 patients die directly or indirectly from colorectal cancer. There is a significant difference in the morbidity rate in various regions, which is closely related to the daily diet, in which the morbidity rate of colorectal cancer in men is higher than that in women. In addition, the morbidity rate of colorectal cancer increases with age. For example, the median age of colorectal cancer in developed countries is 70 years. In the past few decades, the survival rate of colorectal cancer has increased in many countries. Especially in some high-income countries, such as the United States, Australia, Canada and some European countries, the 5-year survival rate of colorectal cancer is over 65%. But relatively, in some low-income countries this value is less than 50%. The expected survival time of colorectal cancer will decrease with the age of onset. The disease stage of colorectal cancer is the most important prognostic factor. For example, between 2001 and 2007, the 5-year survival rates of colorectal cancer patients with different stages in the United States were 90.1% (stage I), 69.2% (stage II and III), and 11.7% (stage IV), respectively.

Colorectal cancer is mainly diagnosed through histological specimens taken by endoscopy. 2% -4% of patients will be forced to undergo a complete colonoscopy or a CT colonography after the diagnosis of colorectal cancer to exclude other concurrent tumors. For rectal cancer, a precise local staging during the diagnosis is necessary, and is also an important basis for the neoadjuvant therapy. In addition to the exact distance to the anal opening, the extent of tumor invasion is also important. As a non-invasive examination method, ultrasound endoscopy can distinguish whether the tumor has infiltrated. Therefore, ultrasound endoscopy is one of the options for local tumor staging. However, due to the effects of radiation during the neoadjuvant treatment, the inspection results of any method are not 100% reliable. These diagnostic techniques have not greatly reduced the mortality rate of colorectal cancer patients. Therefore, how to improve the survival rate of colorectal cancer patients relies on the early diagnosis of colorectal cancer, and the screening and mining of valuable early colorectal cancer biomarkers have become an urgent problem to be solved. Because imaging technology failed to show good results in the early screening of colorectal cancer, people began to turn their attention to molecular markers for the early diagnosis of colorectal cancer. Unfortunately, so far, no molecular marker with high sensitivity and specificity has been found. In recent years, research on colorectal cancer epigenetics has made rapid progress, especially in DNA methylation. It has been found that many specific tumor-related genes have different degrees of methylation status change in the early stage of colorectal cancer, which provides an opportunity for the exploration of markers for early diagnosis of colorectal cancer.

Abnormal DNA methylation of the genome and the occurrence of tumors have always been one of the hotspots in medical research. Cell cycle, DNA repair, angiogenesis, apoptosis, etc. all involve the methylation of related genes. The most likely regulatory role of DNA hypermethylation is to suppress the expression of key genes, thereby determining the fate of a cell. For example, the study of abnormal DNA methylation in tumor cells has made many significant advances in various tumors. In mammals, methylation only affects a cytosine in front of a guanine (CpG) on a DNA strand. The methylation distribution of CpG dinucleotides in normal cells is not uniform. About 50% of genes have CpG islands with concentrated distribution of CpGs in the promoter region, with lengths ranging from 0.5 to 2kb. This region is closely related to gene transcription regulation. In humans, the methylation of CpG islands in certain gene regulatory regions occurs frequently in relevant cancer cell tissues, showing a correlation with the onset, disease progression, prognosis, drug sensitivity, ect. of certain tumors. To date, gene methylation abnormalities have been found in most human tumors. Studies have found that epigenetic coding in cancer cells is disturbed, first of all manifested in the disturbance of DNA methylation level, also known as methylation rearrangement. Since the local hypermethylation of a CpG island in a tumor suppressor gene is earlier than the malignant proliferation of cells, the detection of DNA methylation can be used for the early diagnosis of tumorigenesis. Methylation of cancer-related genes is also an early event of colorectal cancer, so the methylation status of related genes has become an effective indicator for the risk prediction of early colorectal cancer. Even so, there is still a lack of means to effectively detect the methylation status of these cancer-related genes and process the detected results.

WO 2011/126768 A2 discloses a method for detecting colorectal tumors, comprising detecting the level of methylation of marker genes in a sample, wherein the marker genes comprise ALX4, bmp-3, vimentin, septin9, NDRG4 and others.

RU 2 630 669 C1 discloses a method for detecting the methylation of colorectal cancer marker genes, the marker genes comprising ADHFE1, ALX4, SDC2, VIM and others.

Rasmussen Simon L. et al. "Hypermethylated DNA, a circulating biomarker for colorectal cancer detection",PLOS ONE,12(7), 10 July 2017, page e0180809 discloses hypermethylated promoter regions (ALX4, BMP3,

NPTX2, RARB, SDC2, SEPT9, and VIM), with the ability to detect colorectal cancer.

At present, what is urgently needed in the field of gastrointestinal oncology is a clinical test that mini-invasively evaluates and predicts the presence of a colorectal cancer.

SUMMARY

In order to solve the above problems, in one aspect, a method for identifying a colorectal cancer status in a subject is provided herein, which comprises the following steps: 1) detecting methylation levels of biomarker genes in a biological sample from the subject, wherein the biomarker genes comprise: ALX4, BCAT1, IKZF1, NPTX2 and VIM; and 2) comparing the methylation levels detected in step 1) with normal methylation levels of the corresponding biomarker genes in a population to determine the colorectal cancer status in the subject.

In some embodiments, step 1) may comprise extracting DNA from the biological sample and treating the extracted DNA with bisulfite, so that unmethylated cytosine residues in the DNA are deaminated, and methylated cytosine residues remain unchanged.

In some preferred embodiments, the bisulfite is sodium bisulfite.

In some preferred embodiments, in step 1) the biomarker genes comprise ALX4, BCAT1, BMP3, IKZF1, NDRG4, NPTX2, RARB, SDC2, Septin9 and VIM.

In some embodiments, step 1) comprises detecting the methylation levels of a target region within the biomarker genes, wherein the target region is a nucleotide sequence of at least 15 bases in the biomarker genes, or a complementary sequence thereof.

In some embodiments, the detection of the methylation level of the ALX4 gene comprises the use of a primer pair having the sequences as set forth in SEQ ID NOs: 11 and 12 or a primer pair having the sequences as set forth in SEQ ID NOs: 15 and 16 to carry out a PCR amplification reaction, with the bisulfite-treated ALX4 gene or a fragment thereof in the biological sample as a template; the detection of the methylation level of the BCAT1 gene comprises the use of a primer pair having the sequences as set forth in SEQ ID NOs: 19 and 20 or a primer pair having the sequences as set forth in SEQ ID NOs: 23 and 24 to carry out a PCR amplification reaction, with the bisulfite-treated BCAT1 gene or a fragment thereof in the biological sample as a template; the detection of the methylation level of the BMP3 gene comprises the use of a primer pair having the sequences as set forth in SEQ ID NOs: 27 and 28 or a primer pair having the sequences as set forth in SEQ ID NOs: 31 and 32 to carry out a PCR amplification reaction, with the bisulfite-treated BMP3 gene or a fragment thereof in the biological sample as a template; the detection of the methylation level of the IKZF1 gene comprises the use of a primer pair having the sequences as set forth in SEQ ID NOs: 35 and 36 or a primer pair having the sequences as set forth in SEQ ID NOs: 39 and 40 to carry out a PCR amplification reaction, with the bisulfite-treated IKZF1 gene or a fragment thereof in the biological sample as a template; the detection of the methylation level of the NDRG4 gene comprises the use of a primer pair having the sequences as set forth in SEQ ID NOs: 43 and 44 or a primer pair having the sequences as set forth in SEQ ID NOs: 47 and 48 to carry out a PCR amplification reaction, with the bisulfite-treated NDRG4 gene or a fragment thereof in the biological sample as a template;the detection of the methylation level of the NPTX2 gene comprises the use of a primer pair having the sequences as set forth in SEQ ID NOs: 51 and 52, a primer pair having the sequences as set forth in SEQ ID NOs: 55 and 56 or a primer pair having the sequences as set forth in SEQ ID NOs: 59 and 60 to carry out a PCR amplification reaction, with the bisulfite-treated NPTX2 gene or a fragment thereof in the biological sample as a template;the detection of the methylation level of the RARB gene comprises the use of a primer pair having the sequences as set forth in SEQ ID NOs: 63 and 64, a primer pair having the sequences as set forth in SEQ ID NOs: 67 and 68 or a primer pair having the sequences as set forth in SEQ ID NOs: 71 and 72 to carry out a PCR amplification reaction, with the bisulfite-treated RARB gene or a fragment thereof in the biological sample as a template;the detection of the methylation level of the SDC2 gene comprises the use of a primer pair having the sequences as set forth in SEQ ID NOs: 75 and 76, a primer pair having the sequences as set forth in SEQ ID NOs: 79 and 80 or a primer pair having the sequences as set forth in SEQ ID NOs: 83 and 84 to carry out a PCR amplification reaction, with the bisulfite-treated SDC2 gene or a fragment thereof in the biological sample as a template;the detection of the methylation level of the Septin9 gene comprises the use of a primer pair having the sequences as set forth in SEQ ID NOs: 87 and 88 or a primer pair having the sequences as set forth in SEQ ID NOs: 91 and 92 to carry out a PCR amplification reaction, with the bisulfite-treated Septin9 gene or a fragment thereof in the biological sample as a template; and the detection of the methylation level of the VIM gene comprises the use of a primer pair having the sequences as set forth in SEQ ID NOs: 95 and 96 or a primer pair having the sequences as set forth in SEQ ID NOs: 99 and 100 to carry out a PCR amplification reaction, with the bisulfite-treated VIM gene or a fragment thereof in the biological sample as a template.

In some preferred embodiments, the detection of the methylation level of the ALX4 gene comprises the use of a primer pair having the sequences as set forth in SEQ ID NOs: 11 and 12 and a blocking primer having the sequence as set forth in SEQ ID NO: 13, or a primer pair having the sequences as set forth in SEQ ID NOs: 15 and 16 and a blocking primer having the sequence as set forth in SEQ ID NO: 17 to carry out a PCR amplification reaction, with the bisulfite-treated ALX4 gene or a fragment thereof in the biological sample as a template; the detection of the methylation level of the BCAT1 gene

comprises the use of a primer pair having the sequences as set forth in SEQ ID NOs: 19 and 20 and a blocking primer having the sequence as set forth in SEQ ID NO: 21, or a primer pair having the sequences as set forth in SEQ ID NOs: 23 and 24 and a blocking primer having the sequence as set forth in SEQ ID NO: 25 to carry out a PCR amplification reaction, with the bisulfite-treated BCAT1 gene or a fragment thereof in the biological sample as a template; the detection of the methylation level of the BMP3 gene comprises the use of a primer pair having the sequences as set forth in SEQ ID NOs: 27 and 28 and a blocking primer having the sequence as set forth in SEQ ID NO: 29, or a primer pair having the sequences as set forth in SEQ ID NOs: 31 and 32 and a blocking primer having the sequence as set forth in SEQ ID NO: 33 to carry out a PCR amplification reaction, with the bisulfite-treated BMP3 gene or a fragment thereof in the biological sample as a template; the detection of the methylation level of the IKZF1 gene comprises the use of a primer pair having the sequences as set forth in SEQ ID NOs: 35 and 36 and a blocking primer having the sequence as set forth in SEQ ID NO: 37, or a primer pair having the sequences as set forth in SEQ ID NOs: 39 and 40 and a blocking primer having the sequence as set forth in SEQ ID NO: 41 to carry out a PCR amplification reaction, with the bisulfite-treated IKZF1 gene or a fragment thereof in the biological sample as a template; the detection of the methylation level of the NDRG4 gene comprises the use of a primer pair having the sequences as set forth in SEQ ID NOs: 43 and 44 and a blocking primer having the sequence as set forth in SEQ ID NO: 45, or a primer pair having the sequences as set forth in SEQ ID NOs: 47 and 48 and a blocking primer having the sequence as set forth in SEQ ID NO: 49 to carry out a PCR amplification reaction, with the bisulfite-treated NDRG4 gene or a fragment thereof in the biological sample as a template; the detection of the methylation level of the NPTX2 gene comprises the use of a primer pair having the sequences as set forth in SEQ ID NOs: 51 and 52 and a blocking primer having the sequence as set forth in SEQ ID NO: 53, a primer pair having the sequences as set forth in SEQ ID NOs: 55 and 56 and a blocking primer having the sequence as set forth in SEQ ID NO: 57,or a primer pair having the sequences as set forth in SEQ ID NOs: 59 and 60 and a blocking primer having the sequence as set forth in SEQ ID NO: 61 to carry out a PCR amplification reaction, with the bisulfite-treated NPTX2 gene or a fragment thereof in the biological sample as a template; the detection of the methylation level of the RARB gene comprises the use of a primer pair having the sequences as set forth in SEQ ID NOs: 63 and 64 and a blocking primer having the sequence as set forth in SEQ ID NO: 65, a primer pair having the sequences as set forth in SEQ ID NOs: 67 and 68 and a blocking primer having the sequence as set forth in SEQ ID NO: 69, or a primer pair having the sequences as set forth in SEQ ID NOs: 71 and 72 and a blocking primer having the sequence as set forth in SEQ ID NO: 73 to carry out a PCR amplification reaction, with the bisulfite-treated RARB gene or a fragment thereof in the biological sample as a template; the detection of the methylation level of the SDC2 gene comprises the use of a primer pair having the sequences as set forth in SEQ ID NOs: 75 and 76 and a blocking primer having the sequence as set forth in SEQ ID NO: 77, a primer pair having the sequences as set forth in SEQ ID NOs: 79 and 80 and a blocking primer having the sequence as set forth in SEQ ID NO: 81, or a primer pair having the sequences as set forth in SEQ ID NOs: 83 and 84 and a blocking primer having the sequence as set forth in SEQ ID NO: 85 to carry out a PCR amplification reaction, with the bisulfite-treated SDC2 gene or a fragment thereof in the biological sample as a template; the detection of the methylation level of the Septin9 gene comprises the use of a primer pair having the sequences as set forth in SEQ ID NOs: 87 and 88 and a blocking primer having the sequence as set forth in SEQ ID NO: 89, or a primer pair having the sequences as set forth in SEQ ID NOs: 91 and 92 and a blocking primer having the sequence as set forth in SEQ ID NO: 93 to carry out a PCR amplification reaction, with the bisulfite-treated Septin9 gene or a fragment thereof in the biological sample as a template; and the detection of the methylation level of the VIM gene comprises the use of a primer pair having the sequences as set forth in SEQ ID NOs: 95 and 96 and a blocking primer having the sequence as set forth in SEQ ID NO: 97, or a primer pair having the sequences as set forth in SEQ ID NOs: 99 and 100 and a blocking primer having the sequence as set forth in SEQ ID NO: 101 to carry out a PCR amplification reaction, with the bisulfite-treated VIM gene or a fragment thereof in the biological sample as a template, wherein the blocking primers have a 3' end modification which prevents the extension and amplification of a DNA polymerase.

In further preferred embodiments, the detection of the methylation level of the ALX4 gene in step 2) comprises the use of a primer pair having the sequences as set forth in SEQ ID NOs: 11 and 12, a blocking primer having the sequence as set forth in SEQ ID NO: 13 and a probe having the sequence as set forth in SEQ ID NO: 14; or a primer pair having the sequences as set forth in SEQ ID NOs: 15 and 16, a blocking primer having the sequence as set forth in SEQ ID NO: 17 and a probe having the sequence as set forth in SEQ ID NO: 18 to carry out a PCR amplification reaction, with the bisulfite-treated ALX4 gene or a fragment thereof in the biological sample as a template; the detection of the methylation level of the BCAT1 gene comprises the use of a primer pair having the sequences as set forth in SEQ ID NOs: 19 and 20, a blocking primer having the sequence as set forth in SEQ ID NO: 21 and a probe having the sequence as set forth in SEQ ID NO: 22; or a primer pair having the sequences as set forth in SEQ ID NOs: 23 and 24, a blocking primer having the sequence as set forth in SEQ ID NO: 25 and a probe having the sequence as set forth in SEQ ID NO: 26 to carry out a PCR amplification reaction, with the bisulfite-treated BCAT1 gene or a fragment thereof in the biological sample as a template; the detection of the methylation level of the BMP3 gene comprises the use of a primer pair having the sequences as set forth in SEQ ID NOs: 27 and 28, a blocking primer having the sequence as set forth in SEQ ID NO: 29 and a probe having the sequence as set forth in SEQ ID NO: 14; or a primer pair having the sequences as set forth in SEQ ID NOs: 31 and 32, a blocking primer having the sequence as set forth in SEQ ID NO: 33 and a probe having the sequence as set forth in SEQ ID NO: 34 to carry

out a PCR amplification reaction, with the bisulfite-treated BMP3 gene or a fragment thereof in the biological sample as a template; the detection of the methylation level of the IKZF1 gene comprises the use of a primer pair having the sequences as set forth in SEQ ID NOs: 35 and 36, a blocking primer having the sequence as set forth in SEQ ID NO: 37 and a probe having the sequence as set forth in SEQ ID NO: 38; or a primer pair having the sequences as set forth in SEQ ID NOs: 39 and 40, a blocking primer having the sequence as set forth in SEQ ID NO: 41 and a probe having the sequence as set forth in SEQ ID NO: 42 to carry out a PCR amplification reaction, with the bisulfite-treated IKZF1 gene or a fragment thereof in the biological sample as a template; the detection of the methylation level of the NDRG4 gene comprises the use of a primer pair having the sequences as set forth in SEQ ID NOs: 43 and 44, a blocking primer having the sequence as set forth in SEQ ID NO: 45 and a probe having the sequence as set forth in SEQ ID NO: 46, or a primer pair having the sequences as set forth in SEQ ID NOs: 47 and 48, a blocking primer having the sequence as set forth in SEQ ID NO: 49 and a probe having the sequence as set forth in SEQ ID NO: 50 to carry out a PCR amplification reaction, with the bisulfite-treated NDRG4 gene or a fragment thereof in the biological sample as a template; the detection of the methylation level of the NPTX2 gene comprises the use of a primer pair having the sequences as set forth in SEQ ID NOs: 51 and 52, a blocking primer having the sequence as set forth in SEQ ID NO: 53 and a probe having the sequence as set forth in SEQ ID NO: 54; a primer pair having the sequences as set forth in SEQ ID NOs: 55 and 56, a blocking primer having the sequence as set forth in SEQ ID NO: 57 and a probe having the sequence as set forth in SEQ ID NO:58; or a primer pair having the sequences as set forth in SEQ ID NOs: 59 and 60, a blocking primer having the sequence as set forth in SEQ ID NO: 61 and a probe having the sequence as set forth in SEQ ID NO: 62 to carry out a PCR amplification reaction, with the bisulfite-treated NPTX2 gene or a fragment thereof in the biological sample as a template; the detection of the methylation level of the RARB gene comprises the use of a primer pair having the sequences as set forth in SEQ ID NOs: 63 and 64, a blocking primer having the sequence as set forth in SEQ ID NO: 65 and a probe having the sequence as set forth in SEQ ID NO: 66; a primer pair having the sequences as set forth in SEQ ID NOs: 67 and 68, a blocking primer having the sequence as set forth in SEQ ID NO: 69 and a probe having the sequence as set forth in SEQ ID NO: 70; or a primer pair having the sequences as set forth in SEQ ID NOs: 71 and 72, a blocking primer having the sequence as set forth in SEQ ID NO: 73 and a probe having the sequence as set forth in SEQ ID NO: 74 to carry out a PCR amplification reaction, with the bisulfite-treated RARB gene or a fragment thereof in the biological sample as a template; the detection of the methylation level of the SDC2 gene comprises the use of a primer pair having the sequences as set forth in SEQ ID NOs: 75 and 76, a blocking primer having the sequence as set forth in SEQ ID NO: 77 and a probe having the sequence as set forth in SEQ ID NO: 78; a primer pair having the sequences as set forth in SEQ ID NOs: 79 and 80, a blocking primer having the sequence as set forth in SEQ ID NO: 81 and a probe having the sequence as set forth in SEQ ID NO: 82; or a primer pair having the sequences as set forth in SEQ ID NOs: 83 and 84, a blocking primer having the sequence as set forth in SEQ ID NO: 85 and a probe having the sequence as set forth in SEQ ID NO: 86 to carry out a PCR amplification reaction, with the bisulfite-treated SDC2 gene or a fragment thereof in the biological sample as a template; the detection of the methylation level of the Septin9 gene comprises the use of a primer pair having the sequences as set forth in SEQ ID NOs: 87 and 88, a blocking primer having the sequence as set forth in SEQ ID NO: 89 and a probe having the sequence as set forth in SEQ ID NO: 90; or a primer pair having the sequences as set forth in SEQ ID NOs: 91 and 92, a blocking primer having the sequence as set forth in SEQ ID NO: 93 and a probe having the sequence as set forth in SEQ ID NO: 94 to carry out a PCR amplification reaction, with the bisulfite-treated Septin9 gene or a fragment thereof in the biological sample as a template; and the detection of the methylation level of the VIM gene comprises the use of a primer pair having the sequences as set forth in SEQ ID NOs: 95 and 96, a blocking primer having the sequence as set forth in SEQ ID NO: 97 and a probe having the sequence as set forth in SEQ ID NO: 98; or a primer pair having the sequences as set forth in SEQ ID NOs: 99 and 100, a blocking primer having the sequence as set forth in SEQ ID NO: 101 and a probe having the sequence as set forth in SEQ ID NO: 102 to carry out a PCR amplification reaction, with the bisulfite-treated VIM gene or a fragment thereof in the biological sample as a template, wherein the probes have a fluorescent group at one end and a fluorescence quenching group at the other end.

In some embodiments, step 1) further comprises using a primer pair having the sequences as set forth in SEQ ID NOs: 103 and 104 and a probe having the sequence as set forth in SEQ ID NO: 105 to carry out a PCR amplification reaction, with a bisulfite-treated ACTB gene or a fragment thereof used as an internal reference gene in the biological sample as a template.

In some embodiments, step 2) comprises determining the colorectal cancer status in the subject according to the methylation levels of the biomarker gene(s) based on a logistic regression.

In another aspect, a kit for identifying a colorectal cancer status in a subject is provided herein, which comprises primer pairs for detecting methylation levels of biomarker genes in a biological sample from the subject, wherein the primer pairs are used to carry out a PCR amplification reaction with a bisulfite-treated biomarker genes or fragments thereof which are bisulfite-treated as templates; and the biomarker genes comprise ALX4, BCAT1, IKZF1, NPTX2 and VIM.

In some preferred embodiments, the biomarker genes comprise ALX4, BCAT1, BMP3, IKZF1, NDRG4, NPTX2, RARB, SDC2, Septin9 and VIM.

In some embodiments, in the kit, the primer pair used for the the detection of the methylation level of ALX4 has the sequences as set forth in SEQ ID NOs: 11 and 12 or has the sequences as set forth in SEQ ID NOs: 15 and 16; the primer

pair used for the the detection of the methylation level of BCAT1 has the sequences as set forth in SEQ ID NOs: 19 and 20 or has the sequences as set forth in SEQ ID NOs: 23 and 24; the primer pair used for the the detection of the methylation level of BMP3 has the sequences as set forth in SEQ ID NOs: 27 and 28 or has the sequences as set forth in SEQ ID NOs: 31 and 32; the primer pair used for the the detection of the methylation level of IKZF1 has the sequences as set forth in SEQ ID NOs: 35 and 36 or has the sequences as set forth in SEQ ID NOs: 39 and 40; the primer pair used for the the detection of the methylation level of NDRG4 has the sequences as set forth in SEQ ID NOs: 43 and 44 or has the sequences as set forth in SEQ ID NOs: 47 and 48; the primer pair used for the the detection of the methylation level of NPTX2 has the sequences as set forth in SEQ ID NOs: 51 and 52, has the sequences as set forth in SEQ ID NOs: 55 and 56 or has the sequences as set forth in SEQ ID NOs: 59 and 60; the primer pair used for the the detection of the methylation level of RARB has the sequences as set forth in SEQ ID NOs: 63 and 64, has the sequences as set forth in SEQ ID NOs: 67 and 68, or has the sequences as set forth in SEQ ID NOs: 71 and 72; the primer pair used for the the detection of the methylation level of SDC2 has the sequences as set forth in SEQ ID NOs: 75 and 76, has the sequences as set forth in SEQ ID NOs: 79 and 80 or has the sequences as set forth in SEQ ID NOs: 83 and 84; the primer pair used for the the detection of the methylation level of Septin9 has the sequences as set forth in SEQ ID NOs: 87 and 88 or has the sequences as set forth in SEQ ID NOs: 91 and 92; and the primer pair used for the the detection of the methylation level of VIM has the sequences as set forth in SEQ ID NOs: 95 and 96 or has the sequences as set forth in SEQ ID NOs: 99 and 100.

In preferred embodiments, the kit may further comprises a blocking primer, wherein the blocking primer used in combination with the primer pair having the sequences as set forth in SEQ ID NO: 11 and 12 has the sequence as set forth in SEQ ID NO: 13; the blocking primer used in combination with the primer pair having the sequences as set forth in SEQ ID NO: 15 and 16 has the sequence as set forth in SEQ ID NO: 17; the blocking primer used in combination with the primer pair having the sequences as set forth in SEQ ID NO: 19 and 20 has the sequence as set forth in SEQ ID NO: 21; the blocking primer used in combination with the primer pair having the sequences as set forth in SEQ ID NO: 23 and 24 has the sequence as set forth in SEQ ID NO: 25; the blocking primer used in combination with the primer pair having the sequences as set forth in SEQ ID NO: 27 and 28 has the sequence as set forth in SEQ ID NO: 29; the blocking primer used in combination with the primer pair having the sequences as set forth in SEQ ID NO: 31 and 32 has the sequence as set forth in SEQ ID NO: 33; the blocking primer used in combination with the primer pair having the sequences as set forth in SEQ ID NO: 35 and 36 has the sequence as set forth in SEQ ID NO: 37; the blocking primer used in combination with the primer pair having the sequences as set forth in SEQ ID NO: 39 and 40 has the sequence as set forth in SEQ ID NO: 41; the blocking primer used in combination with the primer pair having the sequences as set forth in SEQ ID NO: 43 and 44 has the sequence as set forth in SEQ ID NO: 45; the blocking primer used in combination with the primer pair having the sequences as set forth in SEQ ID NO: 47 and 48 has the sequence as set forth in SEQ ID NO: 49; the blocking primer used in combination with the primer pair having the sequences as set forth in SEQ ID NO: 51 and 52 has the sequence as set forth in SEQ ID NO: 53; the blocking primer used in combination with the primer pair having the sequences as set forth in SEQ ID NO: 55 and 56 has the sequence as set forth in SEQ ID NO: 57; the blocking primer used in combination with the primer pair having the sequences as set forth in SEQ ID NO: 59 and 60 has the sequence as set forth in SEQ ID NO: 61; the blocking primer used in combination with the primer pair having the sequences as set forth in SEQ ID NO: 63 and 64 has the sequence as set forth in SEQ ID NO: 65; the blocking primer used in combination with the primer pair having the sequences as set forth in SEQ ID NO: 67 and 68 has the sequence as set forth in SEQ ID NO: 69; the blocking primer used in combination with the primer pair having the sequences as set forth in SEQ ID NO: 71 and 72 has the sequence as set forth in SEQ ID NO: 73; the blocking primer used in combination with the primer pair having the sequences as set forth in SEQ ID NO: 75 and 76 has the sequence as set forth in SEQ ID NO: 77; the blocking primer used in combination with the primer pair having the sequences as set forth in SEQ ID NO: 79 and 80 has the sequence as set forth in SEQ ID NO: 81; the blocking primer used in combination with the primer pair having the sequences as set forth in SEQ ID NO: 83 and 84 has the sequence as set forth in SEQ ID NO: 85; the blocking primer used in combination with the primer pair having the sequences as set forth in SEQ ID NO: 87 and 88 has the sequence as set forth in SEQ ID NO: 89; the blocking primer used in combination with the primer pair having the sequences as set forth in SEQ ID NO: 91 and 92 has the sequence as set forth in SEQ ID NO: 93; the blocking primer used in combination with the primer pair having the sequences as set forth in SEQ ID NO: 95 and 96 has the sequence as set forth in SEQ ID NO: 97; and the blocking primer used in combination with the primer pair having the sequences as set forth in SEQ ID NO: 99 and 100 has the sequence as set forth in SEQ ID NO: 101, wherein the blocking primers have a 3' end modification which prevents the extension and amplification of a DNA polymerase.

In preferred embodiments, the kit may further comprises a probe, wherein the probe used in combination with the primer pair having the sequences as set forth in SEQ ID NO: 11 and 12 has the sequence as set forth in SEQ ID NO: 14; the probe used in combination with the primer pair having the sequences as set forth in SEQ ID NO: 15 and 16 has the sequence as set forth in SEQ ID NO: 18; the probe used in combination with the primer pair having the sequences as set forth in SEQ ID NO: 19 and 20 has the sequence as set forth in SEQ ID NO: 22; the probe used in combination with the primer pair having the sequences as set forth in SEQ ID NO: 23 and 24 has the sequence as set forth in SEQ ID NO: 26; the probe used in combination with the primer pair having the sequences as set forth in SEQ ID NO: 27 and 28 has the sequence as set forth in SEQ ID NO: 30; the probe used in combination with the primer pair having the sequences as set

forth in SEQ ID NO: 31 and 32 has the sequence as set forth in SEQ ID NO: 34; the probe used in combination with the primer pair having the sequences as set forth in SEQ ID NO: 35 and 36 has the sequence as set forth in SEQ ID NO: 38; the probe used in combination with the primer pair having the sequences as set forth in SEQ ID NO: 39 and 40 has the sequence as set forth in SEQ ID NO: 42; the probe used in combination with the primer pair having the sequences as set forth in SEQ ID NO: 43 and 44 has the sequence as set forth in SEQ ID NO: 46; the probe used in combination with the primer pair having the sequences as set forth in SEQ ID NO: 47 and 48 has the sequence as set forth in SEQ ID NO: 50; the probe used in combination with the primer pair having the sequences as set forth in SEQ ID NO: 51 and 52 has the sequence as set forth in SEQ ID NO: 54; the probe used in combination with the primer pair having the sequences as set forth in SEQ ID NO: 55 and 56 has the sequence as set forth in SEQ ID NO: 58; the probe used in combination with the primer pair having the sequences as set forth in SEQ ID NO: 59 and 60 has the sequence as set forth in SEQ ID NO: 62; the probe used in combination with the primer pair having the sequences as set forth in SEQ ID NO: 63 and 64 has the sequence as set forth in SEQ ID NO: 66; the probe used in combination with the primer pair having the sequences as set forth in SEQ ID NO: 67 and 68 has the sequence as set forth in SEQ ID NO: 70; the probe used in combination with the primer pair having the sequences as set forth in SEQ ID NO: 71 and 72 has the sequence as set forth in SEQ ID NO: 74; the probe used in combination with the primer pair having the sequences as set forth in SEQ ID NO: 75 and 76 has the sequence as set forth in SEQ ID NO: 78; the probe used in combination with the primer pair having the sequences as set forth in SEQ ID NO: 79 and 80 has the sequence as set forth in SEQ ID NO: 82; the probe used in combination with the primer pair having the sequences as set forth in SEQ ID NO: 83 and 84 has the sequence as set forth in SEQ ID NO: 86; the probe used in combination with the primer pair having the sequences as set forth in SEQ ID NO: 87 and 88 has the sequence as set forth in SEQ ID NO: 90; the probe used in combination with the primer pair having the sequences as set forth in SEQ ID NO: 91 and 92 has the sequence as set forth in SEQ ID NO: 94; the probe used in combination with the primer pair having the sequences as set forth in SEQ ID NO: 95 and 96 has the sequence as set forth in SEQ ID NO: 98; and the probe used in combination with the primer pair having the sequences as set forth in SEQ ID NO: 99 and 100 has the sequence as set forth in SEQ ID NO: 102, wherein the probes have a fluorescent group at one end and a fluorescence quenching group at the other end.

In further preferred embodiments, the kit comprises the primer pair and the corresponding blocking primer and probe.

In some embodiments, the kit further comprises a primer pair having the sequences as set forth in SEQ ID NOs: 103 and 104 and a probe having the sequence as set forth in SEQ ID NO: 105, for carrying out a PCR amplification reaction, with a bisulfite-treated ACTB gene or a fragment thereof used as an internal reference gene in the biological sample as a template.

In preferred embodiments, the kit further comprises a DNA extraction reagent and a bisulfite reagent. Preferably, the bisulfite reagent comprises sodium bisulfite.

In preferred embodiments, the kit further comprises an instruction that discribes how to use the kit and process detection results with a logistic regression.

The colorectal cancer status includes the colorectal cancer susceptibility and the presence, progression, subtype, and/or stage of the colorectal cancer.

The biological sample is selected from blood, serum, plasma, feces, lymph, cerebrospinal fluid, ascite, urine, and tissue biopsy from the subject.

The method and kit provided by the present application provide a fast, reliable, and accurate new way for the prediction, diagnosis, and evaluation of a colorectal cancer.

BRIEF DESCRIPTION OF THE DRAWINGS

Figure 1 shows the receiver operating characteristic (ROC) curves of the methylation levels of 10 biomarker genes.

Figure 2 shows the methylation level distribution of 10 biomarker genes in different colorectal cancer stages. Figure 2A shows the methylation level distribution of ALX4, BCAT1, BMP3, IKZF1, NDRG4, and NPTX2, and Figure 2B shows the methylation level distribution of RARB, SDC2, Septin9 and VIM.

Figure 3 shows the methylation level distribution of 10 biomarker genes in different colorectal cancer subtypes. Figure 3A shows the methylation level distribution of ALX4, BCAT1, BMP3, IKZF1, NDRG4, and NPTX2, and Figure 2B shows the methylation level distribution of RARB, SDC2, Septin9 and VIM.

Figure 4 shows the receiver operating characteristic (ROC) curve of a logistic regression model constructed with 10 marker genes;

Figure 5 shows the receiver operating characteristic (ROC) curve of a logistic regression model constructed with the five most characteristic marker genes.

DETAILED DESCRIPTION

Unless otherwise stated, the technical terms used in this application have the meanings generally understood by those skilled in the art to which the present invention belongs.

The present application in one aspect relates to a method for identifying a colorectal cancer status in a subject, which comprises the following steps: 1) detecting the methylation levels of biomarker genes in a biological sample, wherein the biomarker genes comprise ALX4 (Aristaless-Like Homeobox 4), BCAT1 (Branched Chain Amino acid Transaminase 1), IKZF1 (IKAROS Family Zinc Finger 1), NPTX2 (Neuronal pentraxin 2) and VIM (Vimentin); and 2) comparing the methylation levels detected in step 1) with the normal methylation levels of the corresponding biomarker genes in a population to determine the colorectal cancer status in the subject.

The term "subject" as used herein refers to an individual (preferably a human) suffering from or suspected of having a certain disease, or, when predicting the susceptibility, "subject" may also include healthy individuals. The term is generally used interchangeably with "patient", "test subject", "treatment subject", and the like.

The term "population" as used herein generally refers to healthy people. When referring to a specific disease (such as a colorectal cancer), a "population" may include individuals who do not suffer from the specific disease but may suffer from other diseases. In addition, it is also possible to select only some individuals as the "population" based on characteristics such as, age, gender, health status, smoking or not, etc. A "normal methylation level in a population" can be obtained by detecting enough individuals or can be found in an existing clinical literature. In some cases, this normal level refers to no methylation.

The term "colorectal cancer status" used herein includes a colorectal cancer susceptibility and the presence, progression, subtype, and/or stage of a colorectal cancer. In some embodiments, the subject’s susceptibility to a colorectal cancer can be predicted based on the methylation levels of the biomarker gene(s) in the subject. In other embodiments, the subject may be identified for the presence of a colorectal cancer based on the methylation levels of the biomarker gene(s) in the subject; and if a colorectal cancer is present, the subtype and/or the stage of the colorectal cancer may be identified. Colorectal cancer subtypes may include adenocarcinoma, mucoid carcinoma, undifferentiated carcinoma, and other colorectal cancers. The colorectal cancer stages may include stage I (IA, IB, or IC), stage II, stage III, and stage IV. In some embodiments, the colorectal cancer is a stage I colorectal cancer. In some embodiments, the colorectal cancer is a stage II colorectal cancer. In some embodiments, the colorectal cancer is a stage III colorectal cancer. In other embodiments, the colorectal cancer is a stage IV colorectal cancer.

In the method treatment of the subject, for example, including performing more tests on the subject, performing a surgery, giving medications, and taking no further actions, may also be arranged based on the stage of the colorectal cancer. In other embodiments, the method of the present invention further comprises measuring the methylation levels of one or more biomarker genes of ALX4, BCAT1, BMP3, IKZF1, NDRG4, NPTX2, RARB, SDC2, Septin9 and VIM genes or fragments thereof in the subject after the subject is treated, and correlating the measurement results with the colorectal cancer status to identify whether the treatment results in a change in the colorectal cancer status in the subject. In some embodiments, the correlation is performed by a classification algorithm of a software.

The detection of the methylation levels in step 1) comprises extracting DNA from a biological sample, treating it with bisulfite, and then carrying out a PCR amplification by using a methylation-specific primer pair. The bisulfite treatment causes unmethylated cytosine residues in a double-stranded DNA molecule to deaminate to be uracils; while methylated cytosine residues remain unchanged. As a result, in the subsequent PCR amplification reaction, methylated cytosine residue sites on a template are paired with guanine residues in a primer as cytosine residues, while unmethylated cytosine residue sites are paired with adenine residues in a primer as uracil residues. The inventors designed multiple primer pairs for each biomarker gene to detect the methylation level of a target region within each biomarker gene. The target regions are selected from the fragments of at least 15 consecutive bases in the sequences as set forth in SEQ ID NOs: 1-10, respectively; and the nucleic acid sequences of the primer pairs are respectively identical, complementary or hybridizable to the above target regions. The primer pairs provided herein make use of the methylation difference to detect the methylation levels of the target regions within the biomarker genes. When a target region of a biomarker gene is not methylated, the primer pair used cannot effectively pair with and bind to the target region (treated with bisulfite) which is used as a template in the PCR amplification reaction, and cannot (or rarely) generate amplification products; and when the target gene of the biomarker gene is methylated, the primer pair used is able to effectively pair with and bind to the target region (treated with bisulfite) which is used as a template in the PCR amplification reaction, and thus generate amplification products. The differences of these amplification reactions can be monitored in real time during the amplification reactions, or can be judged by detecting the amplification products. After many experiments, the inventors screened out multiple primer pairs for the biomarker genes (see below), which can be used alone or in combination to help identify the colorectal cancer status in the subject.

The term "biomarker gene or a fragment thereof" is often used herein when referring to the detection of a methylation level, because, in the choice of a template, as long as the length of the template is not less than the length of the region to be amplified, the primer pair used in the PCR amplification reaction does not distinguish between the entire gene

or a fragment thereof (in fact, during the DNA extraction and subsequent bisulfite treatment, the gene is usually broken into fragments of different sizes).

In some preferred embodiments, the present invention uses the HeavyMethyl method to measure marker gene methylation. Therefore, in addition to the design of common Taqman primers, blocking primers are further designed. The nucleotide sequence of a blocking primer is designed to be paired with and bind to a template sequence in the region amplified by a corresponding primer pair. In addition, a chemical modification is introduced into a blocking primer at 3'-OH, which prevents the amplification with a DNA polymerase. The chemical modifications are, for example, C3 spacer (C3 Spacer), C6 spacer (C6 Spacer), inverted 3'end , 3' phosphate (3'P), etc. In embodiments of the method of the present invention, the nucleotide sequence of a blocking primer is designed to bind to an unmethylated template (treated with sulfite), but not to a methylated template (treated with sulfite). Therefore, when no methylation occurs in the region corresponding to a blocking primer, it can prevent the corresponding amplification reaction, and thereby improving the specificity of the detection method of the present invention.

In further preferred embodiments of the method of the present invention, it also comprises the use of fluorescent probes to monitor and/or quantify PCR amplification reactions in real time. The fluorescent report group at 5' end of a probe used may be FAM, JOE, TET, HEX, Cy3, Texas Red, Rox, or Cy5; the quenching group at the 3' end is BHQ1, BHQ2, BHQ3, TAMRA, DABCYL, or MGB.

The detection of the methylation levels of the biomarker gene(s) in the method of the present invention includes detecting whether there is/are methylation(s) in the biomarker gene, and quantitative and qualitative detection of the methylation(s).

The biological sample is selected from fluids or tissues extracted form the subject, and includes blood, serum, plasma, feces, lymph, cerebrospinal fluid, ascite, urine, tissue biopsy, etc., preferably plasma, serum and feces.

In the method of the present invention, the age of the subject can also be considered to predict the colorectal cancer status in the subject.

In some embodiments, the method of the present invention further comprises the step of providing a written report or an electronic report on the colorectal cancer prediction, and optionally, the report comprises a prediction about the presence or not or likelihood of a colorectal cancer in the subject, or about the risk gradation of a colorectal cancer in the subject.

In some embodiments, the method of the present invention also comprises establishing a report for a physician on the relative methylation levels of biomarker gene(s), and transmitting such report by post, fax, mailbox, etc. In one embodiment, a data stream containing the report of methylation levels of biomarker gene(s) is transmitted through the internet.

In some embodiments, a statistical method is used to construct a diagnostic model based on the methylation levels of the biomarker gene(s). The statistical method is selected from the following methods: multiple linear regression, lookup table, decision tree, support vector machine, Probit regression, logistic regression, cluster analysis, neighborhood analysis, genetic algorithm, Bayesian and non-Bayesian methods, etc.

In other embodiments, a prediction or diagnostic model based on the methylation levels of the biomarker gene(s) is provided. The model may be in the form of software code, a computer-readable format, or a written description for evaluating the relative methylation levels of the biomarker gene(s).

New and important additional information, which assists the physician in grading the risk of a patient suffering from a colorectal cancer and planning the diagnostic steps to be taken next, can be obtained by using the method of the present invention. The method provided herein can similarly be used to assess the risk of a colorectal cancer in an asymptomatic high-risk patient, and as a screening tool for the general population. It is contemplated that the method of the present invention can be used by a clinician as part of a comprehensive assessment of other predictive and diagnostic indicators.

The method of the present invention can be used to evaluate the therapeutic efficacies of existing chemotherapeutic agents, candidate chemotherapeutic agents and other types of cancer treatments. For example, biological samples can be taken from a subject before or after a treatment or during a treatment of the subject, and the methylation levels of the biomarker gene(s) can be detected as described above. The detection results are used to identify changes in the cancer status in the subject so as to determine the therapeutic efficacy.

The method of the invention can also be used to identify whether a subject is potentially developing a cancer. Relative methylation levels of the biomarker gene(s) in biological samples taken from a subject over time are detected, and the changes in the methylation levels of the biomarkers that point to the characteristics of a cancer are interpreted as a progress toward the cancer.

The combination of the biomarker genes provides a sensitive, specific and accurate means for predicting the presence of a colorectal cancer or detecting a colorectal cancer in different stages of the colorectal cancer progression. Evaluation of the methylation levels in the biological sample may also be correlated with the presence of a pre-malignant or pre-clinical disorder in a patient. Therefore, the disclosed method can be used to predict or detect the presence of a colorectal cancer in a sample, the stage of a colorectal cancer, the subtype of a colorectal cancer, the benignity or

malignancy of a colorectal cancer, the possibility of metastasis of a colorectal cancer, the histological type of a neoplasm associated with a colorectal cancer, the painlessness or aggressiveness of a cancer, and other colorectal cancer characteristics related to the prevention, diagnosis, characterization, and treatment of a colorectal cancer in a patient.

The method of the present invention can also be used to evaluate the effectiveness of candidate drugs to inhibit colorectal cancer, evaluate the efficacy of colorectal cancer therapy, monitor the progress of colorectal cancer, select agents or therapies to inhibit colorectal cancer, monitor the treatment of colorectal cancer patients, monitor the inhibition status of colorectal cancer in patients, and test the methylation levels of biomarker genes in animals after exposure to test compounds to assess the carcinogenic potential of the test compounds.

The present invention also provides a kit for detecting the colorectal cancer status. In some embodiments, the kit may include a DNA extraction reagent and a bisulfite reagent. The DNA extraction reagent may include a lysis buffer, a binding buffer, a washing buffer, and an elution buffer. The lysis buffer is usually composed of a protein denaturant, a detergent, a pH buffering agent and a nuclease inhibitor. The binding buffer is usually composed of a protein denaturant and a pH buffer agent. The washing buffer is divided into washing buffer A and washing buffer B: washing buffer A is composed of a protein denaturant, a nuclease inhibitor, a detergent, a pH buffering agent and ethanol; washing buffer B is composed of a nuclease inhibitor, a pH buffering agent and ethanol. The elution buffer is usually composed of a nuclease inhibitor and a pH buffering agent. The protein denaturant is selected from one or more of guanidine isothiocyanate, guanidine hydrochloride and urea; the detergent is selected from one or more of Tween20, IGEPAL CA-630, Triton X-100, NP-40 and SDS; the pH buffering agent is selected from one or more of Tris, boric acid, phosphate, MES and HEPES; the nuclease inhibitor is selected from one or more of EDTA, EGTA and DEPC. The bisulfite reagents include a bisulfite buffer and a protective buffer, in which the bisulfite salt is selected from one or more of sodium metabisulphite, sodium sulfite, sodium bisulfite, ammonium bisulfite and ammonium sulfite; the protection buffer is composed of an oxygen radical scavenger, and the oxygen radical scavenger is selected from one or more of hydroquinone, vitamin E, vitamin E derivatives, gallic acid, Trolox, trihydroxybenzoic acid and trihydroxybenzoic acid derivatives.

The kit of the present invention comprises primer pairs for methylation-specific PCR amplification reaction(s) for ALX4, BCAT1, IKZF1, NPTX2 and VIM gene. These primer pairs respectively detect the methylation of at least one nucleotide sequence in the nucleotide sequence of a target region of the corresponding gene.

The kit of the present invention may further comprise blocking primers and probes used in combination with the above-mentioned primer pairs (these blocking primers and probes are described above and below).

In certain embodiments, the kit may further comprise an instruction for using the kit to extract DNA from a biological sample and treating the DNA with the bisulfite reagent. In other embodiments, the kit further comprises an instruction for using the reagents in the kit to measure a biomarker level in the subject. In still other embodiments, the kit comprises an instruction for using the kit to determine the colorectal cancer status in a subject.

The present invention also protects the method for detecting the methylation levels of the biomarker genes or fragments thereof with the kit. The method comprises the steps: extracting DNA in a biological sample by using the DNA extraction reagents, treating the extracted DNA with the bisulfite reagents, and using the treated DNA as a template to detect the methylation levels of the biomarker genes with the provided primer pairs.

The measurement method for the methylation level of a biomarker gene may be selected from one or more of the following methods: real-time fluorescent PCR, digital PCR, bisulfite sequencing, methylation-specific PCR, restriction enzyme analysis, high-resolution dissolution curve technology, gene chip technology and time-of-flight mass spectrometry.

The present invention is further described by the following examples.

### Example 1: DNA extraction

The DNA extraction reagent is composed of a lysis buffer, a binding buffer, a washing buffer, and an elution buffer. The lysis buffer is composed of a protein denaturant, a detergent, a pH buffering agent and a nuclease inhibitor. The binding buffer is composed of a protein denaturant and a pH buffering agent. The washing buffer is divided into washing buffer A and washing buffer B. Washing buffer A is composed of a protein denaturant, a nuclease inhibitor, a detergent, a pH buffering agent and ethanol; washing buffer B is composed of a nuclease inhibitor, a pH buffering agent and ethanol. The elution buffer is composed of a nuclease inhibitor and a pH buffering agent. The protein denaturant is guanidine hydrochloride; the detergent is Tween20; the pH buffering agent is Tris-HCl; and the nuclease inhibitor is EDTA.

In this example, a plasma sample of a colorectal cancer patient is taken as an example to extract plasma DNA. The extraction method comprises the following steps:

(1) add to 1 ml of the plasma the same volume of the lysis buffer, then add proteinase K and Carrier RNA to achieve a final concentration of 100 mg/L and 1 μg/ml, mix by shaking, and incubate at 55°C for 30 min;
(2) add 100μl magnetic beads (purchased from Life technologies, catalog No: 37002D), and incubate for 1 hour with shaking;

(3) adsorbe the magnetic beads with a magnetic separator, and discard the supernatant solution;
(4) add 1ml of the washing buffer A to resuspend the magnetic beads and wash for 1min with shaking;
(5) adsorb the magnetic beads with the magnetic separator and discard the supernatant;
(6) add 1ml of washing buffer B to resuspend the magnetic beads and wash for 1min with shaking;
(7) adsorb the magnetic beads with the magnetic separator and discard the supernatant solution;
(8) quickly centrifuge at 10,000 rpm for 1 min, absorb the magnetic beads with the magnetic separator, and remove the residual supernatant solution;
(9) place the centrifuge tube loaded with the magnetic beads on a 55°C metal bath, and dry it for 10 min, with the lid open;
(10) add 100 μl of the elution buffer to resuspend the magnetic beads, place it on a 65°C metal bath, and elute for 10 min with shaking;
(11) adsorb the magnetic beads with the magnetic separator, take out the buffer containing the target DNA, quantify the DNA, and make a mark;
(12) store the eluted DNA in a refrigerator at 4°C for later use, or in a refrigerator at -20°C for long-term storage.

**Example 2: treatment of DNA with bisulfite**

Treatment of DNA with bisulfite is to treat the extracted DNA sample with the bisulfite reagent. The bisulfite reagent is composed of a bisulfite buffer and a protection buffer. The bisulfite buffer is a mixed liquid of sodium bisulfite and water; the protective buffer is a mixed liquid of oxygen radical scavenger hydroquinone and water.

The DNA extracted in Example 1 is used as the processing object in this Example, and the DNA is treated with bisulfite. The specific steps comprise:

(1) prepare the bisulfite buffer: weigh 1 g of sodium bisulfite powder, and add water to it to obtain 3 M buffer solution;
(2) prepare the protection buffer: weigh 1 g of hydroquinone reagent, and add water to it to obtain 0.5M protection buffer;
(3) mix together 100 μl of the DNA solution, 200 μl of the bisulfite buffer and 50 μl of the protection solution, and mix by shaking;
(4) perform a thermal cycling: 95°C for 5 min, 80°C for 60 min, and 4°C for 10 min;
(5) add 1 ml of the DNA binding buffer to the bisulfite-treated DNA solution, add 50 μl magnetic beads, and incubate for 1 h with shaking;
(6) adsorbe the magnetic beads with a magnetic separator, and discard the supernatant solution;
(7) add 0.5ml of the washing buffer A to resuspend the magnetic beads and wash for 1min with shaking;
(8) adsorbe the magnetic beads with the magnetic separator, and discard the supernatant;
(9) add 0.5ml of the washing buffer B to resuspend the magnetic beads and wash for 1min with shaking;
(10) adsorbe the magnetic beads with the magnetic separator, and discard the supernatant;
(11) quickly centrifuge at 10,000 rpm for 1 min, absorb the magnetic beads with the magnetic separator, and remove the residual supernatant solution;
(12) place the centrifuge tube loaded with the magnetic beads on a 55°C metal bath, and dry it for 10 min, with the lid open;
(13) add 50 μl of the elution buffer to resuspend the magnetic beads, place it on a 65°C metal bath, and elute for 10 min with shaking;
(14) adsorb the magnetic beads with the magnetic separator, take out the buffer containing the target DNA, quantify the DNA, and make a mark.

**Example 3: Real-time fluorescent PCR detection of DNA methylation and verification of primer sets**

In this example, a real-time fluorescent PCR was used as an example to detect the methylation levels of biomarker genes. The genes to be detected were ALX4, BCAT1, BMP3, IKZF1, NDRG4, NPTX2, RARB, SDC2, Septin9 and VIM genes, and the internal reference gene was ACTB. In this example, the bisulfite-treated DNA of Example 2 was used as a template for real-time fluorescent PCR amplification. The DNA samples to be detected, negative quality control products, positive quality control products and no template controls were all detected in three replicates.

For ALX4, BCAT1, BMP3, IKZF1, NDRG4, NPTX2, RARB, SDC2, Septin9 and VIM genes, multiple sets of primer and probe combinations could be designed. However, the performance of each set of the probe and primer combinations may be different, so they needed to be verified through experiments.

Therefore, we designed a variety of primers and probes for ALX4, BCAT1, BMP3, IKZF1, NDRG4, NPTX2, RARB, SDC2, Septin9 and VIM genes, which were respectively equivalent to, complementary to, or hybridizable to at least 15 consecutive nucleotides of the sequences as set forth in SEQ ID NOs: 1-10 or complementary sequences thereof, and

verified the effectiveness of the designed primers and probes with methylated and unmethylated nucleic acid sequences as templates. We selected the following optimal primer sets and a primer set for the internal reference gene ACTB through real-time fluorescence PCR amplification results.

ALX4 primer set 1
primer 1: SEQ ID NO 11: 5'- TGCGTAAGTTAGGTATGA -3'
primer 2: SEQ ID NO 12: 5'- CTACGACACCGAACTATA -3'
blocking primer: SEQ ID NO 13: 5'- TGTAAGTTAGGTATGAATGTTGAGATTTGTG -C3-3'
probe: SEQ ID NO 14: 5'-HEX- CCATAACAACGACCGACGACTC -BHQ1-3'
ALX4 primer set 2
primer 1: SEQ ID NO 15: 5'- GTAGGATTGTAGAAGTTTCG -3'
primer 2: SEQ ID NO 16: 5'- TACGCCAATACACCTAAA -3'
blocking primer: SEQ ID NO 17: 5'- GTTTTGTTTTTTGTTGGTTGGGAGG -C3-3'
probe: SEQ ID NO 18: 5'-HEX- CAACCACGCTCCGAACTTCC -BHQ1-3'
BCAT1 primer set 1
primer 1: SEQ ID NO 19: 5'-TGTTGATGTAATTCGTTAGGTCGC-3'
primer 2: SEQ ID NO 20: 5'-AATACCCGAAACGACGACG-3'
blocking primer: SEQ ID NO 21: 5'-ATTTGTTAGGTTGTGAGTTTTTGTTGTGAGAG-C3-3'
probe: SEQ ID NO 22: 5'-Texas Red-AAACCGACCCTCTCGCGACGAA-BHQ2-3'
BCAT1 primer set 2
primer 1: SEQ ID NO 23: 5'- TTTATTGTTTCGTCGGTTACG -3'
primer 2: SEQ ID NO 24: 5'- CCCAAATCTRACTACAACCG -3'
blocking primer: SEQ ID NO 25: 5'- TGTTGGTTATGAGGGAAGTTTGAGTTGAGTG -C3-3'
probe: SEQ ID NO 26: 5'-Texas Red- CGCGCTCTACAACCGCAAACCCG -BHQ2-3'
BMP3 primer set 1
primer 1: SEQ ID NO 27 5'- CGGGTTATATACGTCGC -3'
primer 2: SEQ ID NO 28: 5'- CCAACAACTACGCGAA -3'
blocking primer: SEQ ID NO 29: 5'- TACACAAACCTCACCCACACAAAACACTACA -C3-3'
probe: SEQ ID NO 30: 5'-Texas Red- CGCTACGAACGCCGTCTCCAC -BHQ2-3'
BMP3 primer set 2
primer 1: SEQ ID NO 31: 5'- TTGGGTTAGCGTAGTAAG -3'
primer 2: SEQ ID NO 32: 5'- CCAACTAAAACGAAAACG -3'
blocking primer: SEQ ID NO 33: 5'- TGTAGTAAGTGGGGTTGGTTGTT -C3-3'
probe: SEQ ID NO 34: 5'-Texas Red- CGACCGAATACAACGAAATAACG -BHQ2-3'
IKZF1 primer set 1
primer 1: SEQ ID NO 35: 5'- GTAGGTACGGTTCGTATTC -3'
primer 2: SEQ ID NO 36: 5'- CGCACGAAAACTTTACAA -3'
blocking primer: SEQ ID NO 37: 5'- GTATTTGTCGTTGTTTTGGTGGTTTTTG -C3-3'
probe: SEQ ID NO 38: 5'-FAM- CGCCGAACTCCGACTCAACC -BHQ1-3'
IKZF1 primer set 2
primer 1: SEQ ID NO 39: 5'- GACGGGACGACGTATTTTTTTC -3'
primer 2: SEQ ID NO 40: 5'- CGCGCGCACCTCTCGA -3'
blocking primer: SEQ ID NO 41: 5'- GGGATTGTTAGTGTGTGTTATTTTAAAGT -C3-3'
probe: SEQ ID NO 42: 5'-FAM- CGCCTCCCGAATCGCTACTCCGATAC -BHQ1-3'
NDRG4 primer set 1
primer 1: SEQ ID NO 43: 5'- CGTAGCGTATTTAGTATAGTTC -3'
primer 2: SEQ ID NO 44: 5'- CCGATAAACGAACGAAAA -3'
blocking primer: SEQ ID NO 45: 5'- ATTTAGTATAGTTTGTGTGGTGGAGTG -C3-3'
probe: SEQ ID NO 46: 5'-Texas Red- ACCGCGACGCGAAACCTAAA -BHQ2-3'
NDRG4 primer set 2
primer 1: SEQ ID NO 47: 5'- CGTTCGGGATTAGTTTTAG -3'
primer 2: SEQ ID NO 48: 5'- CCGCGTAAATTTAACGAA -3'
blocking primer: SEQ ID NO 49: 5'- TGGGATTAGTTTTAGGTTTGGTATTG -C3-3'
probe: SEQ ID NO 50: 5'-Texas Red- ACCCGCGAAACGATACCGAA -BHQ2-3'
NPTX2 primer set 1
primer 1: SEQ ID NO 51: 5'- CGTAACGGAAAGCGTTTTCG -3'
primer 2: SEQ ID NO 52: 5'- TACTCGACACTCTAACCTACCGAATC -3'
blocking primer: SEQ ID NO 53 5'- ATGGAAAGTGTTTTTGTTTTGTTTTGTTTTG -C3-3'

probe: SEQ ID NO 54: 5’-JOE- ACCGAATACGCGTCACGCAATAAAC -BHQ1-3’
NPTX2 primer set 2
primer 1: SEQ ID NO 55: 5’- ATTTTCGAGACGATAGCGCG -3’
primer 2: SEQ ID NO 56: 5’- TACACACGAAACGACTACCGAAC -3’
blocking primer: SEQ ID NO 57 5’- TTGAGATGATAGTGTGGTTATTGTTAGTAGTGAA-C3-3’
probe: SEQ ID NO 58: 5’-JOE- TCCGCGAAAAACGCCTTCGCT -BHQ1-3’
NPTX2 primer set 3
primer 1: SEQ ID NO 59: 5’- CGGATTCGGTAGGTTAGA-3’
primer 2: SEQ ID NO 60: 5’- CGCTATCGTCTCGAAAATCG-3’
blocking primer: SEQ ID NO 61 5’- AGGTTAGAGTGTTGAGTAGTGTGGTG-C3-3’
probe: SEQ ID NO 62: 5’-JOE- AATCTCCTACCGTCTCACAACCG-BHQ1-3’
RARB primer set 1
primer 1: SEQ ID NO 63: 5’- GCGTATAGAGGAATTTAAAGTGTGG -3’
primer 2: SEQ ID NO 64: 5’- ACGCCTTTTTATTTACGACGACTTAAC -3’
blocking primer: SEQ ID NO 65: 5’- TTATTTACAACAACTTAACTTAAAAAACAATATTCCACC -C3-3’
probe: SEQ ID NO 66: 5’-HEX- TATTCCGCCTACGCCCGCTCG -BHQ1-3’
RARB primer set 2
primer 1: SEQ ID NO 67: 5’- GAATTTTTTTATGCGAGTTGTTTGAGG -3’
primer 2: SEQ ID NO 68: 5’- TTCCGAATACGTTCCGAATCCTACC -3’
blocking primer: SEQ ID NO 69: 5’- TTATGTGAGTTGTTTGAGGATTGGGATGTTGAG -C3-3’
probe: SEQ ID NO 70: 5’-HEX- AACAAACCCTACTCGAATCGCTCGCG -BHQ1-3’
RARB primer set 3
primer 1: SEQ ID NO 71: 5’- TGGGAATTTTTCGTTTCGGTT -3’
primer 2: SEQ ID NO 72: 5’- ACACGTAAACTATTAATCTTTTTCCCAAC -3’
blocking primer: SEQ ID NO 73: 5’- CATAAACTATTAATCTTTTTCCCAACCCCAAATC-C3-3’
probe: SEQ ID NO 74: 5’-HEX- TCATTTACCATTTTCCAAACTTACTCGACC -BHQ1-3’
SDC2 primer set 1
primer 1: SEQ ID NO 75: 5’- CGGCGTAGTTATAGCGCGG -3’
primer 2: SEQ ID NO 76: 5’- CCGAACTCCCCTAAACGACTAAA -3’
blocking primer: SEQ ID NO 77: 5’- AGTTATAGTGTGGAGTTGTGGTGTTTATTGGTT -C3-3’
probe: SEQ ID NO 78: 5’-FAM- TACAAAATTACACGCCGATTAACAACTCCG -BHQ1-3’
SDC2 primer set 2
primer 1: SEQ ID NO 79: 5’- CGTAGGAGGAGGAAGCG -3’
primer 2: SEQ ID NO 80: 5’- GCACACGAATCCGAAAC -3’
blocking primer: SEQ ID NO 81: 5’- GGAGGAAGTGAGTGTTTTTGAGTTTTGAG -C3-3’
probe: SEQ ID NO 82: 5’-FAM- AATACCGCAACGATTACGACTCAAACTCG -BHQ1-3’ SDC2 primer set 3
primer 1: SEQ ID NO 83: 5’- CGAGTTTGAGTCGTAATCGTTG -3’
primer 2: SEQ ID NO 84: 5’- CAACCAAAACAAAACGAAACC -3’
blocking primer: SEQ ID NO 85: 5’- TGTAATTGTTGTGGTATTTTGTTTTGGATTTGTG -C3-3’
probe: SEQ ID NO 86: 5’-FAM- AACGCTCGACGCAACCCGCGC -BHQ1-3’
Septin9 primer and probe combination 1
primer 1: SEQ ID NO 87: 5’- CGCGATTCGTTGTTTATTAG-3’
primer 2: SEQ ID NO 88: 5’- CACCTTCGAAATCCGAAA-3
blocking primer: SEQ ID NO 89: 5’- AAAATCCAAAATAATCCCATCCAACTACACATTAAC -C3-3’
probe: SEQ ID NO 90: 5’-FAM- CGCGTTAACCGCGAAATCCGACATAAT-BHQ1-3’
Septin9 primer and probe combination 2
primer 1: SEQ ID NO 91: 5’- TAGCGTATTTTCGTTTCGC-3’
primer 2: SEQ ID NO 92: 5’-CGAACTTCGAAAATAAATACTAAAC-3
blocking primer: SEQ ID NO 93: 5’- TTTGTTTTGTGTTAGGTTTATTTGTAGGGTTT-C3-3’
probe: SEQ ID NO 94: 5’-FAM-AACTACTACGACCGCGAACGTA-BHQ1-3’
VIM primer set 1
primer 1: SEQ ID NO 95: 5’ TCGTCGTCGTTTAGGTTATCGTTAT -3’
primer 2: SEQ ID NO 96: 5’- ACGAATAAACGTAATCACGTAACTCC -3’
blocking primer: SEQ ID NO 97: 5’- GTTGTTTAGGTTATTGTTATTTTTTGTAGTTATGTTTATT -C3-3’
probe: SEQ ID NO 98: 5’-HEX- TACGATAAAAAAACGAAAACACGAACCTAATAAAC -BHQ1-3’
VIM primer set 2
primer 1: SEQ ID NO 99: 5’ GTCGTAGTTTTTACGTTTCGTTTTT -3’
primer 2: SEQ ID NO 100: 5’- TACGAATATTCTTAAACTCGATATTAATAAC -3’

blocking primer: SEQ ID NO 101: 5'- TTATGTTTTGTTTTTGGGTGGTGTGTATGT -C3-3'
probe: SEQ ID NO 102: 5'-HEX- AACACGCTACTCCGCAAACGC -BHQ1-3'
internal reference gene ACTB primer set
primer 1: SEQ ID NO 103: 5'-GTGATGGAGGAGGTTTAGTAAGT-3'
primer 2: SEQ ID NO 104: 5'-CCAATAAAACCTACTCCTCCCTT-3'
probe: SEQ ID NO 105: 5'-Cy5-ACCACCACCCAACACACAATAACAAACACA-BHQ3-3'

All of the multiple sets of primers and probes could distinguish between methylated and unmethylated templates, and could be used as primers and probes to detect the methylations of ALX4, BCAT1, BMP3, IKZF1, NDRG4, NPTX2, RARB, SDC2, Septin9 and VIM genes, respectively. Although the effectiveness of different primer and probe combinations were slightly different, the above primers and probes were suitable for the detection of methylations of ALX4, BCAT1, BMP3, IKZF1, NDRG4, NPTX2, RARB, SDC2, Septin9 and VIM genes, respectively. Table 1 below showed the detection results of methylated and unmethylated templates (treated with bisulfite) of the above genes with various primer and probe combinations. Obviously, the designed primer and probe combinations were highly specific for the methylated templates.

Table1 detection results of the designed primer sets on methylated and unmethylated templates (Ct, mean)

| | ALX4-1 | ALX4-2 | BCAT1-1 | BCAT1-2 | BMP3-1 | BMP3-2 | IKZF1-1 | IKZF1-2 | NDRG4-1 | NDRG4-2 | NPTX2-1 |
|---|---|---|---|---|---|---|---|---|---|---|---|
| methylated DNA | 33.55 | 33.97 | 35.68 | 35.23 | 36.88 | 37.12 | 35.67 | 31.61 | 32.53 | 34.12 | 30.24 |
| unmethylated DNA | No Ct | No Ct | No Ct | No Ct | No Ct | No Ct | No Ct | No Ct | No Ct | No Ct | No Ct |

| | NPTX2-2 | NPTX2-3 | RARB-1 | RARB-2 | RARB-3 | SDC2-1 | SDC2-2 | SDC2-3 | Septin9-1 | Septin9-2 | VIM-1 | VIM-2 |
|---|---|---|---|---|---|---|---|---|---|---|---|---|
| methylated DNA | 33.11 | 32.08 | 29.81 | 32.67 | 33.18 | 27.89 | 30.34 | 31.97 | 30.32 | 33.02 | 29.89 | 32.08 |
| unmethylated DNA | No Ct | No Ct | No Ct | No Ct | No Ct | No Ct | No Ct | No Ct | No Ct | No Ct | No Ct | No Ct |

Furthermore, we used DNAs from different cancer patients and healthy people as templates to further verify the effectiveness of the primer and probe combinations. DNAs in plasma samples from 5 cases of colorectal cancer, 3 cases of liver cancer, and 5 cases of healthy persons were extracted by using the DNA extraction method of Example 1, and then DNA templates were treated with bisulfite by using the method of Example 2. Using the above-mentioned multiple primer and probe sets, real-time fluorescent PCR experiments were performed. The Ct values of various marker genes in cancer samples and healthy person samples were measured respectively. The results were shown in Table 2.

Table 2 detection results of the methylation levels of the specified genes in individuals with known colorectal cancer status (including healthy individuals) with each primer set.

| | ALX4-1 | ALX4-2 | BCAT1-1 | BCAT1-2 | BMP3-1 | BMP3-2 | IKZF1-1 | IKZF1-2 | NDRG4-1 | NDRG4-2 | NPTX2-1 |
|---|---|---|---|---|---|---|---|---|---|---|---|
| CRC 1 | 33.88 | 36.06 | 36.01 | 37.13 | 28.77 | 38.42 | 31.51 | 38.52 | 31.17 | 36.82 | 36.82 |
| CRC 2 | 34.28 | 36.17 | 35.11 | 37.49 | 31.45 | 36.06 | 28.19 | 36.63 | 32.58 | 35.33 | 35.33 |
| CRC 3 | 35.35 | 36.25 | 30.99 | 37.99 | 36.42 | 38.81 | 36.74 | 38.35 | 35.28 | 37.98 | 30.98 |
| CRC 4 | 35.42 | 35.62 | 31.34 | 35.14 | 33.35 | 36.62 | 34.81 | 37.54 | 33.53 | 37.84 | 33.84 |
| CRC 5 | 34.84 | 36.69 | 36.91 | 35.72 | 32.39 | 38.71 | 36.09 | 38.43 | 34.62 | 36.73 | 34.73 |
| HeCa 1 | 43.46 | 41.35 | 43.64 | 42.25 | No Ct | No Ct | No Ct | 44.42 | 43.86 | 42.25 | 42.25 |
| HeCa 2 | 43.32 | 43.01 | No Ct | 43.07 | No Ct | No Ct | 42.57 | No Ct | No Ct | 42.36 | No Ct |
| HeCa 3 | 42.41 | 42.93 | No Ct | 44.23 | 43.67 | No Ct | No Ct | 44.78 | 43.23 | No Ct | 42.67 |
| Con 1 | No Ct | No Ct | No Ct | No Ct | 43.99 | No Ct | No Ct | 44.42 | No Ct | 44.57 | No Ct |
| Con 2 | No Ct | 44.34 | 44.12 | No Ct | No Ct | No Ct | No Ct | No Ct | No Ct | No Ct | No Ct |
| Con 3 | No Ct | No Ct | 43.89 | 44.44 | No Ct | No Ct | No Ct | No Ct | No Ct | No Ct | 43.27 |
| Con 4 | No Ct | 44.34 | No Ct | No Ct | No Ct | No Ct | No Ct | 44.01 | No Ct | No Ct | No Ct |
| Con 5 | No Ct | No Ct | 44.23 | No Ct | No Ct | No Ct | No Ct | No Ct | 44.36 | No Ct | No Ct |

| | NPTX2-2 | NPTX2-3 | RARB-1 | RARB-2 | RARB-3 | SDC2-1 | SDC2-2 | SDC2-3 | Septin9-1 | Septin9-2 | VIM-1 | VIM-2 |
|---|---|---|---|---|---|---|---|---|---|---|---|---|
| CRC 1 | 35.38 | 36.61 | 31.41 | 35.36 | 36.17 | 33.23 | 38.16 | 36.32 | 32.27 | 36.25 | 32.27 | 36.17 |
| CRC 2 | 34.28 | 36.77 | 33.11 | 37.62 | 35.35 | 34.06 | 34.19 | 35.89 | 33.58 | 36.13 | 33.58 | 35.18 |
| CRC 3 | 35.15 | 35.57 | 32.94 | 36.49 | 36.41 | 32.81 | 35.34 | 37.35 | 33.18 | 35.18 | 33.18 | 36.84 |
| CRC 4 | 32.34 | 33.46 | 31.31 | 36.64 | 35.21 | 35.62 | 35.15 | 36.34 | 34.13 | 36.54 | 34.13 | 33.64 |
| CRC 5 | 31.38 | 34.87 | 33.92 | 33.72 | 32.34 | 32.31 | 34.19 | 37.33 | 34.42 | 34.33 | 34.42 | 36.72 |
| HeCa 1 | 44.56 | 40.67 | 43.64 | 44.15 | 44.14 | No Ct | 43.23 | 44.12 | 43.16 | 42.25 | 43.16 | 44.61 |
| HeCa 2 | 41.58 | 42.15 | No Ct | 42.57 | 43.23 | No Ct | 44.53 | 43.24 | No Ct | No Ct | No Ct | 42.34 |
| HeCa 3 | 43.37 | 43.91 | No Ct | 44.12 | 43.67 | 43.67 | No Ct | 44.28 | No Ct | 43.55 | No Ct | 43.76 |
| Con 1 | No Ct | No Ct | No Ct | No Ct | 44.09 | No Ct | No Ct | 44.12 | No Ct | No Ct | No Ct | No Ct |
| Con 2 | No Ct | 44.33 | No Ct | No Ct | No Ct | No Ct | 44.26 | No Ct | No Ct | 43.99 | No Ct | No Ct |
| Con 3 | 43.51 | No Ct | 43.89 | 44.44 | No Ct | No Ct | No Ct | 43.76 | No Ct | No Ct | No Ct | 44.63 |
| Con 4 | No Ct | No Ct | No Ct | No Ct | No Ct | No Ct | 43.56 | 44.21 | No Ct | No Ct | No Ct | No Ct |
| Con 5 | No Ct | No Ct | 44.23 | No Ct | No Ct | No Ct | No Ct | No Ct | No Ct | 44.16 | No Ct | 43.73 |

abbreviations: CRC:colorectal cancer; HeCa:liver cancer; Con:healthy

As can be seen from the above detected Ct values of the methylations of ALX4, BCAT1, BMP3, IKZF1, NDRG4, NPTX2, RARB, SDC2, Septin9 and VIM genes, each of the above primer and probe combination generated a highly specific amplification for methylated DNA of colorectal cancers, while there were no amplification or the Ct values of the amplifications were greater than 40 for other cancers or the healthy persons. Although the Ct values of the amplifications for colorectal cancer samples with different combinations of primer pair and probe showed some differences, they were obviously different from those of other cancers and healthy person samples. Therefore, all of the above primer sets were suitable for colorectal cancer detection.

**Example 4: sensitivity and specificity of the kit for detecting the plasmas of colorectal cancer patients and benign patients**

296 samples from patients with pathologically identified colorectal cancer and 353 samples from patients with pathologically identified benign disease were used (see Table 3). All of the samples were collected from the Naval General Hospital of People's Liberation Army. The colorectal cancer samples included all stages and common subtypes of the disease. The colorectal cancer patients were confirmed by imaging and pathological diagnosis. The sample staging was based on international TNM staging standards, and the sample subtyping was determined according to tissue biopsies and immunohistochemical methods. Benign samples included common types of benign disorders founded in the whole study population. Complete clinical pathology reports were obtained after surgeries, including patient's age, smoking history, race, stage, subtype, and the collection sites were encoded for each sample.

Table 3 colorectal cancer stages and other characteristics of the collected samples

| | colorectal cancer = stage and subtype | | | | | benign |
|---|---|---|---|---|---|---|
| | I | II | III | IV | total | |
| number of samples(%) | | | | | | - |
| colorectal cancer | | | | | | - |
| adenocarcinoma | 15 | 41 | 115 | 54 | 225(76.1) | - |
| mucoid carcinoma | 4 | 7 | 18 | 8 | 37(12.5) | - |
| undifferentiated carcinoma | 3 | 4 | 5 | 3 | 15(5.1) | - |
| colorectal cancer of other type | 3 | 5 | 9 | 2 | 19(6.4) | - |
| sum | 25 | 57 | 147 | 67 | 296 | - |
| | (8.4) | (19.3) | (49.7) | (22.6) | (100) | - |
| benign | | | | | | |
| polyp | - | - | - | - | - | 132(37.4) |
| hemorrhoid | - | - | - | - | - | 116(32.9) |

(continued)

| | colorectal cancer = stage and subtype | | | | | benign |
|---|---|---|---|---|---|---|
| | I | II | III | IV | total | |
| enteritis | - | - | - | - | - | 57(16.1) |
| no abnormalities | - | - | - | - | - | 48(13.6) |
| total | | | | | | 353(100) |
| ages of the polulation | | | | | | |
| median age (years) | 56 | 62 | 62 | 65 | 62 | 50 |
| age range (years) | 21-85 | 23-88 | 29-95 | 25-90 | 21-95 | 18-85 |
| mean age (years) | 58.3 | 64.2 | 61.9 | 63.7 | 62.3 | 50.1 |
| SD | 13.5 | 10.1 | 8.8 | 11.7 | 9.2 | 8.5 |

DNAs were extracted from the samples by using the DNA extraction method of Example 1, the DNA templates were then treated with bisulfite by using the method of Example 2, and, next, real-time fluorescent PCR experiments were performed with the primer and probe combinations provided in Example 3 (for each biomarker gene, primer set 1 was used) to detect ALX4, BCAT1 BMP3, IKZF1, NDRG4, NPTX2, RARB, SDC2, Septin9 and VIM genes and internal reference gene ACTB, and finally, the Ct values were obtained for each gene from samples of healthy persons and colorectal cancer patients. As described in Example 3 above, the methylation levels of each gene could be indicated by these Ct values.

Commercially available software packages (IBM SPSS Statistics 24 and MedCalc 11.4.2.0, purchased from IBM and MedCalc, respectively) were used for descriptive statistics of plasma biomarker levels, receiver operating characteristic (ROC) curves and graphical displays. The nonparametric Kruskal-Wallis test (ANOVA) was used, and then a Dunn's multiple comparison post-test was used to determine statistical differences. For all statistical comparisons, a P value <0.05 was considered statistically significant.

The methylation levels of the above 10 marker genes were detected in plasmas from 296 patients with pathologically determined colorectal cancer and 353 individuals with benign colorectal disorders by real-time fluorescent PCR assays. To facilitate the determination of the ability of these biomarker genes to distinguish cancers from benign colorectal disorders with similar symptoms, all samples were obtained from the same clinical population (based on patients undergone surgeries for colorectal polyps). All samples were collected before any intervention and before the disease status was known. The disease status was then determined by pathological examination of the isolated tissues. A single sample collection protocol was used to collect the plasmas and compliance was monitored. This ensured sample quality and eliminated any possibility of collection, processing and biological bias in the sample set. Normal healthy samples were not used in this study because they are usually more easily distinguishable than benign disorders. These samples showed that the average patient age among individuals with colorectal cancers (62 years) was higher than that among individuals with benign disorders (50 years), and both increased with the progression of disease stages (Table 3). Overall, the distribution of colorectal cancer subtypes was similar to that found in all colorectal cancer cases in the population, with the proportion (76%) of adenocarcinomas being larger than that of other colorectal cancers. The benign controls in the study represented common benign colorectal diseases, including benign polyps, hemorrhoids, enteritis, etc.

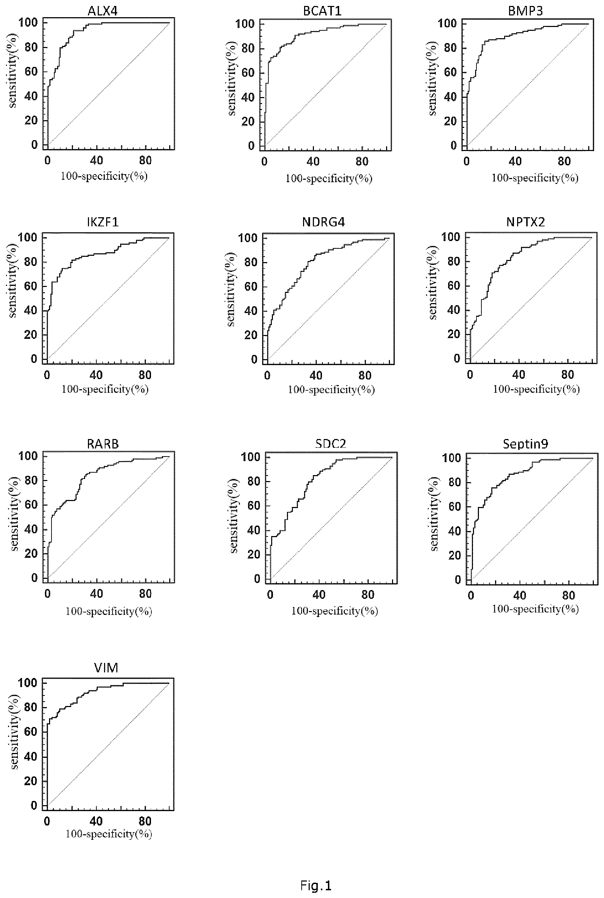

For the detected data of the methylation level of each biomarker, MedCalc 11.4.2.0 software was used to generate a ROC curve and the area under the curve (AUC) value with a 95% confidence interval. Compared with benign colorectal disorders, the AUCs of the methylation levels of 10 biomarker genes in colorectal cancer samples were all greater than 0.8 (P value > 0.05), and ranged from 0.80 to 0.92 (see Figure 1 and Table 4).

Table 4 area under the curves (AUCs) though curve analysis of the receiver operating characteristic (ROC) curves of 10 marker genes

| markers | AUC | standard error | CI |
|---|---|---|---|
| ALX4 | 0.915 | 0.019 | 0.868 to 0.950 |
| BCAT1 | 0.902 | 0.021 | 0.853 to 0.940 |
| BMP3 | 0.838 | 0.027 | 0.779 to 0.886 |
| IKZF1 | 0.872 | 0.018 | 0.817 to 0.915 |

(continued)

| markers | AUC | standard error | CI |
|---|---|---|---|
| NDRG4 | 0.809 | 0.028 | 0.761 to 0.863 |
| NPTX2 | 0.878 | 0.016 | 0.828 to 0.921 |
| RARB | 0.830 | 0.026 | 0.783 to 0.887 |
| SDC2 | 0.822 | 0.028 | 0.762 to 0.872 |
| Septin9 | 0.848 | 0.027 | 0.788 to 0.893 |
| VIM | 0.873 | 0.021 | 0.815 to 0.929 |

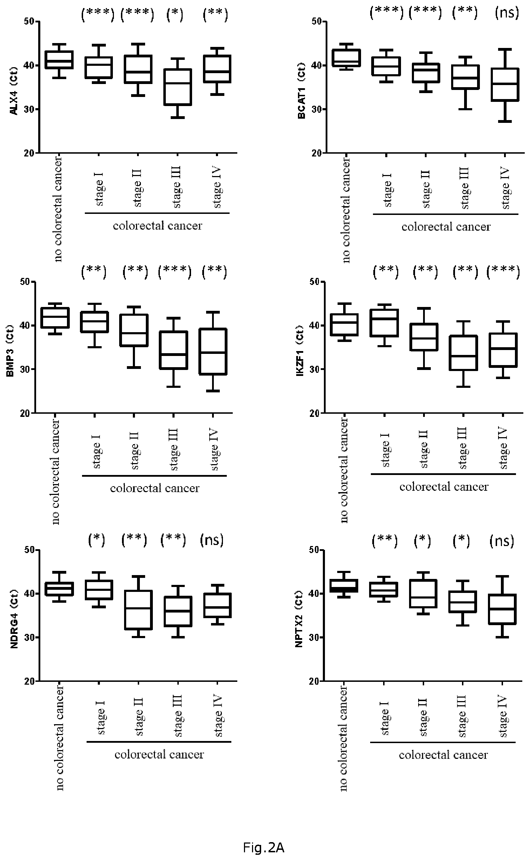

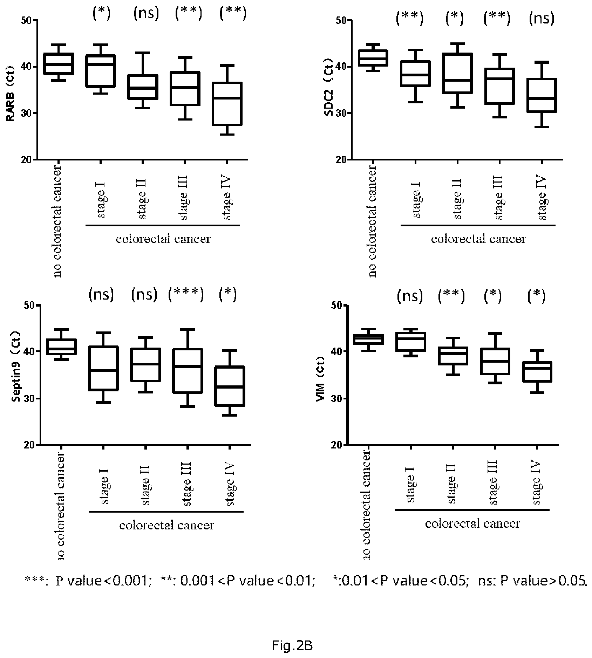

In order to determine whether certain biomarker genes could provide a better distinguishing ability between different stages of colorectal cancers (especially in early stages), the distinguishing abilities of the methylation levels of 10 biomarker genes (Figure 2) in stage I and stage II samples (the most important period for the marker detections) were compared. For stage I samples, both ALX4 and BCAT1 provided very high distinguishing abilities (P value <0.001), followed by BMP3, IKZF1, NPTX2 and SDC2 (P value 0.001 to 0.01) in descending order, and then NDRG4 and RARB (P Value 0.01 to 0.05). For Septin9 and VIM, there were no significant differences between stage I cancers and benign disorders (P value> 0.05). For stage II samples, both ALX4 and BCAT1 again provided very high distinguishing abilities (P value <0.001), followed by BMP3, IKZF1, NDRG4 and VIM (P value 0.001 to 0.01), and then NPTX2 and SDC2 (P value 0.01 to 0.05). There were no significant differences for RARB and Septin9 (P value> 0.05).

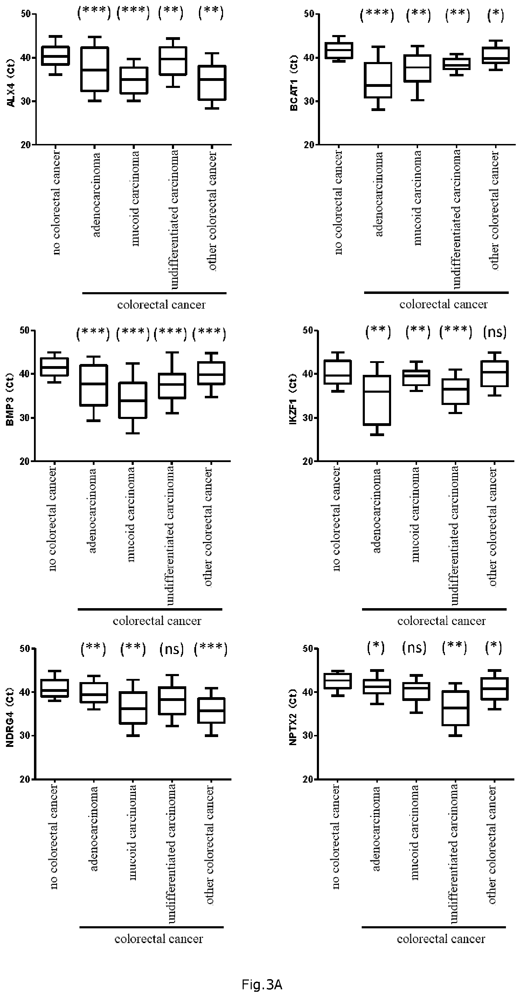

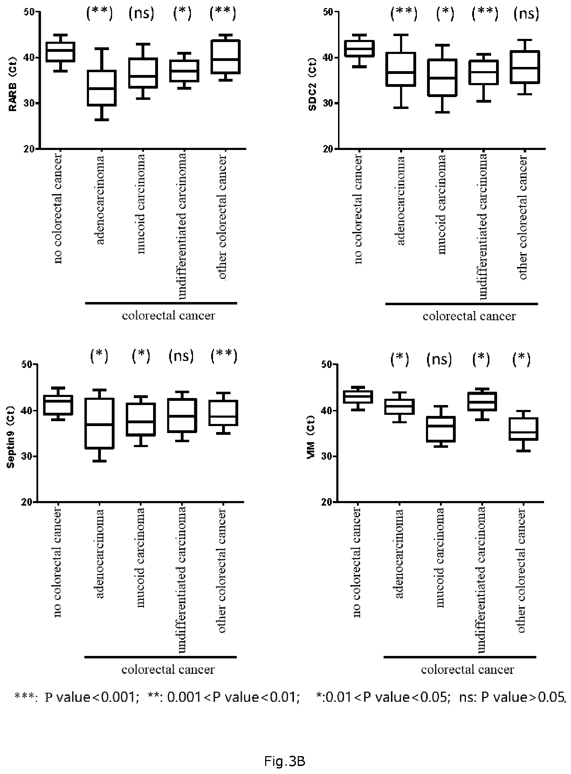

It was also evaluated whether there were statistically significant differences in the methylation levels of the above biomarker genes between samples from benign disorders and various subtypes of colorectal cancers (Figure 3). For adenocarcinomas, both ALX4, BCAT1 and BMP3 provided very high distinguishing abilities (P value <0.001), followed by IKZF1, NDRG4, RARB, SDC2, NPTX2, Septin9 and VIM (P Value 0.001 to 0.05) in descending order. For mucoid carcinomas, ALX4 and BMP3 provided very high distinguishing abilities (P value <0.001), followed by BCAT1, IKZF1, NDRG4, SDC2, and Septin9 (P Value 0.001 to 0.05). For undifferentiated carcinomas, BMP3 and IKZF1 provided very high distinguishing abilities (P value <0.001), followed by ALX4, BCAT1, NPTX2, SDC2, RARB and VIM (P Value 0.001 to 0.05) in descending order. For other colorectal cancers, BMP3 and NDRG4 provided very high distinguishing abilities (P value <0.001), followed by ALX4, RARB, Septin9, BCAT1, NPTX2 and VIM (P Value 0.001 to 0.05) in descending order.

In terms of simple operation and cost reduction, the detection of the methylation level of a single biomarker gene is better than the detection of the methylation levels of multiple biomarker genes. However, it is obvious that the methylation level of a single biomarker gene may not provide information on the inherent diversity of a complex disease, so it is often necessary to establish a diagnostic model with multiple markers. Multi-marker diagnosis model is established by using statistical analysis methods. The establishment of a diagnosis model with methylated gene markers for the detection of colorectal cancers is described below by taking a logistic regression model as an example.

The training of the logistic regression model was conducted as follows: dividing the samples into cases and controls, and then optimizing the regression coefficients with IBM SPSS Statistics 24 software. Maximum likelihood of the data was trained with the logistic regression model by using one regression coefficient for each marker and one deviation parameter.

After training, the regression coefficient set defined the logistic regression model. By putting the methylation levels of the biomarkers into the logistic regression equation, those skilled in the art can easily use such diagnostic model to predict the possibility of any new sample to be identified as a case or a control.

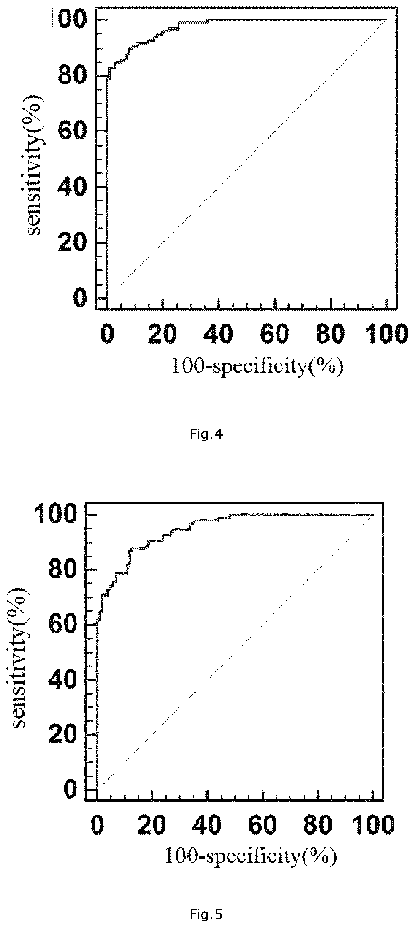

The AUCs of the methylation levels of the above 10 marker genes were all greater than 0.80. Next, we used the logistic regression to combine the 10 marker genes, which generated an AUC of 0.969 (standard error: 0.019; 95% CI: 0.936-0.992; P value: < 0.0001) (Figure 4). In order to simplify the monitoring and analysis method, the five markers with larger AUC values were combined and used to establish a logistic regression model. The obtained AUC value was 0.943 (standard error: 0.017; 95% CI: 0.906-0.976; P value: <0.0001) (Figure 5). For this sample set, a 98.0% sensitivity is acquired at a specificity of 64.1%. Two models were further compared by determining a model’s sensitivity at a fixed specificity value and a model’s specificity at a fixed sensitivity value (see Tables 5 and 6 below). For example, it could be selected that, when the sensitivity of the method was greater than about 95%, the sum of its sensitivity and specificity was greater than about 160%; or when the specificity of the method was greater than about 95%, the sum of its sensitivity and specificity was greater than about 165%. Generally, the sensitivity and specificity of a logistic regression model with 10 markers were slightly better than that with 5 markers. However, when the operational analysis procedures and cost were taken into consideration, the combination of the 5 markers may also be a good choice.

Table 5 sensitivities at important specificity thresholds in logistic regression models of the 5 most characteristic marker genes and of the 10 marker genes

| specificity thresholds(%) | sensitivity(%) | |
|---|---|---|
| | 5 markers | 10 markers |
| 80 | 91.2 | 95.3 |
| 85 | 88.1 | 92.1 |
| 90 | 79.6 | 86.5 |
| 95 | 73.6 | 78.6 |
| 100 | 42.8 | 51.2 |

Table 6 specificities at important sensitivity thresholds in logistic regression models of the 5 most characteristic marker genes and of the 10 marker genes

| sensitivity thresholds(%) | specificity(%) | |
|---|---|---|
| | 5 markers | 10 markers |
| 80 | 89.5 | 94.1 |
| 85 | 87.4 | 93.4 |
| 90 | 81.5 | 79.5 |
| 95 | 71.1 | 79.5 |
| 100 | 52.6 | 58.2 |

It should be noted that the detection results of the methylation levels provided in this Example were obtained with primer set 1 for each biomarker gene (for example, for ALX4 gene, use ALX4 primer set 1; for BCAT1 gene, use BCAT1 primer set 1, and so on), and similar detection results were obtained with other primer sets provided herein (data not shown).

The technical solutions provided by the present invention, through jointly detecting the methylation levels of one or more genes of ALX4, BCAT1, BMP3, IKZF1, NDRG4, NPTX2, RARB, SDC2, Septin9 and VIM genes or fragments thereof, improved the sensitivity and specificity of colorectal cancer detection, and thus ensured the accuracy and reliability of the test results. Moreover, the method for the detection of methylated DNAs of biomarker genes in a sample was able to easily accomplish the detection of the methylation levels of 10 biomarkers: ALX4, BCAT1, BMP3, IKZF1, NDRG4, NPTX2, RARB, SDC2, Septin9and VIM genes, quickly and conveniently determine the sample was positive or not and the risk value by using a logistic regression equation, and provide a kit for rapid detection of the cancer.

## Claims

**1.** A method for identifying a colorectal cancer status in a subject comprising:

1) detecting methylation levels of biomarker genes in a biological sample from the subject, wherein the biomarker genes comprise ALX4, BCAT1, IKZF1, NPTX2 and VIM; and
2) comparing the methylation levels detected in step 1) with normal methylation levels of the corresponding biomarker genes in a population to determine the colorectal cancer status in the subject.

**2.** The method of claim 1, wherein step 1) comprises extracting DNA from the biological sample and treating the extracted DNA with a bisulfite, so that unmethylated cytosine residues in the DNA are deaminated, and methylated cytosine residues remain unchanged.

**3.** The method of claim 1, wherein in step 1) the biomarker genes comprise ALX4, BCAT1, BMP3, IKZF1, NDRG4, NPTX2, RARB, SDC2, Septin9 and VIM.

**4.** The method of claim 1, wherein step 1) comprises detecting the methylation levels of a target region within the biomarker genes, and wherein the target region is a nucleotide sequence of at least 15 bases in the biomarker genes,

or a complementary sequence thereof.

**5.** The method of claim 1, wherein, in step 1),

the detection of the methylation level of the ALX4 gene comprises the use of a primer pair having the sequences as set forth in SEQ ID NOs: 11 and 12 or a primer pair having the sequences as set forth in SEQ ID NOs: 15 and 16 to carry out a PCR amplification reaction, with the ALX4 gene or a fragment thereof which is bisulfite-treated in the biological sample as a template;
the detection of the methylation level of the BCAT1 gene comprises the use of a primer pair having the sequences as set forth in SEQ ID NOs: 19 and 20 or a primer pair having the sequences as set forth in SEQ ID NOs: 23 and 24 to carry out a PCR amplification reaction, with the BCAT1 gene or a fragment thereof which is bisulfite-treated in the biological sample as a template;
the detection of the methylation level of the BMP3 gene comprises the use of a primer pair having the sequences as set forth in SEQ ID NOs: 27 and 28 or a primer pair having the sequences as set forth in SEQ ID NOs: 31 and 32 to carry out a PCR amplification reaction, with the BMP3 gene or a fragment thereof which is bisulfite-treated in the biological sample as a template;
the detection of the methylation level of the IKZF1 gene comprises the use of a primer pair having the sequences as set forth in SEQ ID NOs: 35 and 36 or a primer pair having the sequences as set forth in SEQ ID NOs: 39 and 40 to carry out a PCR amplification reaction, with the IKZF1 gene or a fragment thereof which is bisulfite-treated in the biological sample as a template;
the detection of the methylation level of the NDRG4 gene comprises the use of a primer pair having the sequences as set forth in SEQ ID NOs: 43 and 44 or a primer pair having the sequences as set forth in SEQ ID NOs: 47 and 48 to carry out a PCR amplification reaction, with the NDRG4 gene or a fragment thereof which is bisulfite-treated in the biological sample as a template;
the detection of the methylation level of the NPTX2 gene comprises the use of a primer pair having the sequences as set forth in SEQ ID NOs: 51 and 52, a primer pair having the sequences as set forth in SEQ ID NOs: 55 and 56 or a primer pair having the sequences as set forth in SEQ ID NOs: 59 and 60 to carry out a PCR amplification reaction, with the NPTX2 gene or a fragment thereof which is bisulfite-treated in the biological sample as a template;
the detection of the methylation level of the RARB gene comprises the use of a primer pair having the sequences as set forth in SEQ ID NOs: 63 and 64, a primer pair having the sequences as set forth in SEQ ID NOs: 67 and 68 or a primer pair having the sequences as set forth in SEQ ID NOs: 71 and 72 to carry out a PCR amplification reaction, with the RARB gene or a fragment thereof which is bisulfite-treated in the biological sample as a template;
the detection of the methylation level of the SDC2 gene comprises the use of a primer pair having the sequences as set forth in SEQ ID NOs: 75 and 76, a primer pair having the sequences as set forth in SEQ ID NOs: 79 and 80 or a primer pair having the sequences as set forth in SEQ ID NOs: 83 and 84 to carry out a PCR amplification reaction, with the SDC2 gene or a fragment thereof which is bisulfite-treated in the biological sample as a template;
the detection of the methylation level of the Septin9 gene comprises the use of a primer pair having the sequences as set forth in SEQ ID NOs: 87 and 88 or a primer pair having the sequences as set forth in SEQ ID NOs: 91 and 92 to carry out a PCR amplification reaction, with the Septin9 gene or a fragment thereof which is bisulfite-treated in the biological sample as a template; and
the detection of the methylation level of the VIM gene comprises the use of a primer pair having the sequences as set forth in SEQ ID NOs: 95 and 96 or a primer pair having the sequences as set forth in SEQ ID NOs: 99 and 100 to carry out a PCR amplification reaction, with the VIM gene or a fragment thereof which is bisulfite-treated in the biological sample as a template.

**6.** The method of claim 5, wherein, in step 1),

the detection of the methylation level of the ALX4 gene comprises the use of a primer pair having the sequences as set forth in SEQ ID NOs: 11 and 12 and a blocking primer having the sequence as set forth in SEQ ID NO: 13, or a primer pair having the sequences as set forth in SEQ ID NOs: 15 and 16 and a blocking primer having the sequence as set forth in SEQ ID NO: 17 to carry out a PCR amplification reaction, with the bisulfite-treated ALX4 gene or a fragment thereof in the biological sample as a template;
the detection of the methylation level of the BCAT1 gene comprises the use of a primer pair having the sequences as set forth in SEQ ID NOs: 19 and 20 and a blocking primer having the sequence as set forth in SEQ ID NO: 21, or a primer pair having the sequences as set forth in SEQ ID NOs: 23 and 24 and a blocking primer having the

sequence as set forth in SEQ ID NO: 25 to carry out a PCR amplification reaction, with the bisulfite-treated BCAT1 gene or a fragment thereof in the biological sample as a template;
the detection of the methylation level of the BMP3 gene comprises the use of a primer pair having the sequences as set forth in SEQ ID NOs: 27 and 28 and a blocking primer having the sequence as set forth in SEQ ID NO: 29, or a primer pair having the sequences as set forth in SEQ ID NOs: 31 and 32 and a blocking primer having the sequence as set forth in SEQ ID NO: 33 to carry out a PCR amplification reaction, with the bisulfite-treated BMP3 gene or a fragment thereof in the biological sample as a template;
the detection of the methylation level of the IKZF1 gene comprises the use of a primer pair having the sequences as set forth in SEQ ID NOs: 35 and 36 and a blocking primer having the sequence as set forth in SEQ ID NO: 37, or a primer pair having the sequences as set forth in SEQ ID NOs: 39 and 40 and a blocking primer having the sequence as set forth in SEQ ID NO: 41 to carry out a PCR amplification reaction, with the bisulfite-treated IKZF1 gene or a fragment thereof in the biological sample as a template;
the detection of the methylation level of the NDRG4 gene comprises the use of a primer pair having the sequences as set forth in SEQ ID NOs: 43 and 44 and a blocking primer having the sequence as set forth in SEQ ID NO: 45, or a primer pair having the sequences as set forth in SEQ ID NOs: 47 and 48 and a blocking primer having the sequence as set forth in SEQ ID NO: 49 to carry out a PCR amplification reaction, with the bisulfite-treated NDRG4 gene or a fragment thereof in the biological sample as a template;
the detection of the methylation level of the NPTX2 gene comprises the use of a primer pair having the sequences as set forth in SEQ ID NOs: 51 and 52 and a blocking primer having the sequence as set forth in SEQ ID NO: 53, a primer pair having the sequences as set forth in SEQ ID NOs: 55 and 56 and a blocking primer having the sequence as set forth in SEQ ID NO: 57,or a primer pair having the sequences as set forth in SEQ ID NOs: 59 and 60 and a blocking primer having the sequence as set forth in SEQ ID NO: 61 to carry out a PCR amplification reaction, with the bisulfite-treated NPTX2 gene or a fragment thereof in the biological sample as a template;
the detection of the methylation level of the RARB gene comprises the use of a primer pair having the sequences as set forth in SEQ ID NOs: 63 and 64 and a blocking primer having the sequence as set forth in SEQ ID NO: 65, a primer pair having the sequences as set forth in SEQ ID NOs: 67 and 68 and a blocking primer having the sequence as set forth in SEQ ID NO: 69, or a primer pair having the sequences as set forth in SEQ ID NOs: 71 and 72 and a blocking primer having the sequence as set forth in SEQ ID NO: 73 to carry out a PCR amplification reaction, with the bisulfite-treated RARB gene or a fragment thereof in the biological sample as a template;
the detection of the methylation level of the SDC2 gene comprises the use of a primer pair having the sequences as set forth in SEQ ID NOs: 75 and 76 and a blocking primer having the sequence as set forth in SEQ ID NO: 77, a primer pair having the sequences as set forth in SEQ ID NOs: 79 and 80 and a blocking primer having the sequence as set forth in SEQ ID NO: 81, or a primer pair having the sequences as set forth in SEQ ID NOs: 83 and 84 and a blocking primer having the sequence as set forth in SEQ ID NO: 85 to carry out a PCR amplification reaction, with the bisulfite-treated SDC2 gene or a fragment thereof in the biological sample as a template;
the detection of the methylation level of the Septin9 gene comprises the use of a primer pair having the sequences as set forth in SEQ ID NOs: 87 and 88 and a blocking primer having the sequence as set forth in SEQ ID NO: 89, or a primer pair having the sequences as set forth in SEQ ID NOs: 91 and 92 and a blocking primer having the sequence as set forth in SEQ ID NO: 93 to carry out a PCR amplification reaction, with the bisulfite-treated Septin9 gene or a fragment thereof in the biological sample as a template; and
the detection of the methylation level of the VIM gene comprises the use of a primer pair having the sequences as set forth in SEQ ID NOs: 95 and 96 and a blocking primer having the sequence as set forth in SEQ ID NO: 97, or a primer pair having the sequences as set forth in SEQ ID NOs: 99 and 100 and a blocking primer having the sequence as set forth in SEQ ID NO: 101 to carry out a PCR amplification reaction, with the bisulfite-treated VIM gene or a fragment thereof in the biological sample as a template,
wherein the blocking primers have a 3' end modification which prevents the extension and amplification of a DNA polymerase.

**7.** The method of claim 1, wherein step 2) comprises determining the colorectal cancer status in the subject according to the methylation levels of the biomarker genes based on a logistic regression.

**8.** A kit for identifying a colorectal cancer status in a subject comprising primer pairs for detecting methylation levels of biomarker genes in a biological sample from the subject, wherein the primer pairs are used to carry out a PCR amplification reaction with the biomarker genes or fragments thereof which are bisulfite-treated as templates; and the biomarker genes comprise ALX4, BCAT1, IKZF1, NPTX2 and VIM.

**9.** The kit of claim 8, wherein the biomarker genes comprise ALX4, BCAT1, BMP3, IKZF1, NDRG4, NPTX2, RARB, SDC2, Septin9 and VIM.

**10.** The kit of claim 8, wherein

the primer pair used for the the detection of the methylation level of ALX4 has the sequences as set forth in SEQ ID NOs: 11 and 12 or has the sequences as set forth in SEQ ID NOs: 15 and 16;
the primer pair used for the the detection of the methylation level of BCAT1 has the sequences as set forth in SEQ ID NOs: 19 and 20 or has the sequences as set forth in SEQ ID NOs: 23 and 24;
the primer pair used for the the detection of the methylation level of BMP3 has the sequences as set forth in SEQ ID NOs: 27 and 28 or has the sequences as set forth in SEQ ID NOs: 31 and 32;
the primer pair used for the the detection of the methylation level of IKZF1 has the sequences as set forth in SEQ ID NOs: 35 and 36 or has the sequences as set forth in SEQ ID NOs: 39 and 40;
the primer pair used for the the detection of the methylation level of NDRG4 has the sequences as set forth in SEQ ID NOs: 43 and 44 or has the sequences as set forth in SEQ ID NOs: 47 and 48;
the primer pair used for the the detection of the methylation level of NPTX2 has the sequences as set forth in SEQ ID NOs: 51 and 52, has the sequences as set forth in SEQ ID NOs: 55 and 56 or has the sequences as set forth in SEQ ID NOs: 59 and 60;
the primer pair used for the the detection of the methylation level of RARB has the sequences as set forth in SEQ ID NOs: 63 and 64, has the sequences as set forth in SEQ ID NOs: 67 and 68, or has the sequences as set forth in SEQ ID NOs: 71 and 72;
the primer pair used for the the detection of the methylation level of SDC2 has the sequences as set forth in SEQ ID NOs: 75 and 76, has the sequences as set forth in SEQ ID NOs: 79 and 80 or has the sequences as set forth in SEQ ID NOs: 83 and 84;
the primer pair used for the the detection of the methylation level of Septin9 has the sequences as set forth in SEQ ID NOs: 87 and 88 or has the sequences as set forth in SEQ ID NOs: 91 and 92; and
the primer pair used for the the detection of the methylation level of VIM has the sequences as set forth in SEQ ID NOs: 95 and 96 or has the sequences as set forth in SEQ ID NOs: 99 and 100.

**11.** The kit of claim 10 further comprising a blocking primer, wherein

the blocking primer used in combination with the primer pair having the sequences as set forth in SEQ ID NO: 11 and 12 has the sequence as set forth in SEQ ID NO: 13;
the blocking primer used in combination with the primer pair having the sequences as set forth in SEQ ID NO: 15 and 16 has the sequence as set forth in SEQ ID NO: 17;
the blocking primer used in combination with the primer pair having the sequences as set forth in SEQ ID NO: 19 and 20 has the sequence as set forth in SEQ ID NO: 21;
the blocking primer used in combination with the primer pair having the sequences as set forth in SEQ ID NO: 23 and 24 has the sequence as set forth in SEQ ID NO: 25;
the blocking primer used in combination with the primer pair having the sequences as set forth in SEQ ID NO: 27 and 28 has the sequence as set forth in SEQ ID NO: 29;
the blocking primer used in combination with the primer pair having the sequences as set forth in SEQ ID NO: 31 and 32 has the sequence as set forth in SEQ ID NO: 33;
the blocking primer used in combination with the primer pair having the sequences as set forth in SEQ ID NO: 35 and 36 has the sequence as set forth in SEQ ID NO: 37;
the blocking primer used in combination with the primer pair having the sequences as set forth in SEQ ID NO: 39 and 40 has the sequence as set forth in SEQ ID NO: 41;
the blocking primer used in combination with the primer pair having the sequences as set forth in SEQ ID NO: 43 and 44 has the sequence as set forth in SEQ ID NO: 45;
the blocking primer used in combination with the primer pair having the sequences as set forth in SEQ ID NO: 47 and 48 has the sequence as set forth in SEQ ID NO: 49;
the blocking primer used in combination with the primer pair having the sequences as set forth in SEQ ID NO: 51 and 52 has the sequence as set forth in SEQ ID NO: 53;
the blocking primer used in combination with the primer pair having the sequences as set forth in SEQ ID NO: 55 and 56 has the sequence as set forth in SEQ ID NO: 57;
the blocking primer used in combination with the primer pair having the sequences as set forth in SEQ ID NO: 59 and 60 has the sequence as set forth in SEQ ID NO: 61;
the blocking primer used in combination with the primer pair having the sequences as set forth in SEQ ID NO: 63 and 64 has the sequence as set forth in SEQ ID NO: 65;
the blocking primer used in combination with the primer pair having the sequences as set forth in SEQ ID NO: 67 and 68 has the sequence as set forth in SEQ ID NO: 69;

the blocking primer used in combination with the primer pair having the sequences as set forth in SEQ ID NO: 71 and 72 has the sequence as set forth in SEQ ID NO: 73;
the blocking primer used in combination with the primer pair having the sequences as set forth in SEQ ID NO: 75 and 76 has the sequence as set forth in SEQ ID NO: 77;
the blocking primer used in combination with the primer pair having the sequences as set forth in SEQ ID NO: 79 and 80 has the sequence as set forth in SEQ ID NO: 81;
the blocking primer used in combination with the primer pair having the sequences as set forth in SEQ ID NO: 83 and 84 has the sequence as set forth in SEQ ID NO: 85;
the blocking primer used in combination with the primer pair having the sequences as set forth in SEQ ID NO: 87 and 88 has the sequence as set forth in SEQ ID NO: 89;
the blocking primer used in combination with the primer pair having the sequences as set forth in SEQ ID NO: 91 and 92 has the sequence as set forth in SEQ ID NO: 93;
the blocking primer used in combination with the primer pair having the sequences as set forth in SEQ ID NO: 95 and 96 has the sequence as set forth in SEQ ID NO: 97; and
the blocking primer used in combination with the primer pair having the sequences as set forth in SEQ ID NO: 99 and 100 has the sequence as set forth in SEQ ID NO: 101,
wherein the blocking primers have a 3' end modification which prevents the extension and amplification of a DNA polymerase.

**12.** The kit of claim 11 further comprising a probe, wherein

the probe used in combination with the primer pair having the sequences as set forth in SEQ ID NO: 11 and 12 has the sequence as set forth in SEQ ID NO: 14;
the probe used in combination with the primer pair having the sequences as set forth in SEQ ID NO: 15 and 16 has the sequence as set forth in SEQ ID NO: 18;
the probe used in combination with the primer pair having the sequences as set forth in SEQ ID NO: 19 and 20 has the sequence as set forth in SEQ ID NO: 22;
the probe used in combination with the primer pair having the sequences as set forth in SEQ ID NO: 23 and 24 has the sequence as set forth in SEQ ID NO: 26;
the probe used in combination with the primer pair having the sequences as set forth in SEQ ID NO: 27 and 28 has the sequence as set forth in SEQ ID NO: 30;
the probe used in combination with the primer pair having the sequences as set forth in SEQ ID NO: 31 and 32 has the sequence as set forth in SEQ ID NO: 34;
the probe used in combination with the primer pair having the sequences as set forth in SEQ ID NO: 35 and 36 has the sequence as set forth in SEQ ID NO: 38;
the probe used in combination with the primer pair having the sequences as set forth in SEQ ID NO: 39 and 40 has the sequence as set forth in SEQ ID NO: 42;
the probe used in combination with the primer pair having the sequences as set forth in SEQ ID NO: 43 and 44 has the sequence as set forth in SEQ ID NO: 46;
the probe used in combination with the primer pair having the sequences as set forth in SEQ ID NO: 47 and 48 has the sequence as set forth in SEQ ID NO: 50;
the probe used in combination with the primer pair having the sequences as set forth in SEQ ID NO: 51 and 52 has the sequence as set forth in SEQ ID NO: 54;
the probe used in combination with the primer pair having the sequences as set forth in SEQ ID NO: 55 and 56 has the sequence as set forth in SEQ ID NO: 58;
the probe used in combination with the primer pair having the sequences as set forth in SEQ ID NO: 59 and 60 has the sequence as set forth in SEQ ID NO: 62;
the probe used in combination with the primer pair having the sequences as set forth in SEQ ID NO: 63 and 64 has the sequence as set forth in SEQ ID NO: 66;
the probe used in combination with the primer pair having the sequences as set forth in SEQ ID NO: 67 and 68 has the sequence as set forth in SEQ ID NO: 70;
the probe used in combination with the primer pair having the sequences as set forth in SEQ ID NO: 71 and 72 has the sequence as set forth in SEQ ID NO: 74;
the probe used in combination with the primer pair having the sequences as set forth in SEQ ID NO: 75 and 76 has the sequence as set forth in SEQ ID NO: 78;
the probe used in combination with the primer pair having the sequences as set forth in SEQ ID NO: 79 and 80 has the sequence as set forth in SEQ ID NO: 82;
the probe used in combination with the primer pair having the sequences as set forth in SEQ ID NO: 83 and 84 has

the sequence as set forth in SEQ ID NO: 86;
the probe used in combination with the primer pair having the sequences as set forth in SEQ ID NO: 87 and 88 has the sequence as set forth in SEQ ID NO: 90;
the probe used in combination with the primer pair having the sequences as set forth in SEQ ID NO: 91 and 92 has the sequence as set forth in SEQ ID NO: 94;
the probe used in combination with the primer pair having the sequences as set forth in SEQ ID NO: 95 and 96 has the sequence as set forth in SEQ ID NO: 98; and
the probe used in combination with the primer pair having the sequences as set forth in SEQ ID NO: 99 and 100 has the sequence as set forth in SEQ ID NO: 102,
wherein the probes have a fluorescent group at one end and a fluorescence quenching group at the other end.

**13.** The kit of claim 8, further comprising an instruction that describes how to use the kit and process detection results with a logistic regression.

## Patentansprüche

**1.** Verfahren zur Bestimmung des Darmkrebsstatus bei einer Testperson, umfassend:

1) Nachweisen der Methylierungsgrade von Biomarker-Genen in einer biologischen Probe der Testperson, wobei die Biomarker-Gene ALX4, BCAT1, IKZF1, NPTX2 und VIM umfassen; und
2) Vergleichen der in Schritt 1) nachgewiesenen Methylierungsgrade mit den normalen Methylierungsgraden der entsprechenden Biomarker-Gene in einer Population zum Bestimmen des Darmkrebsstatus bei der Testperson.

**2.** Verfahren nach Anspruch 1, wobei Schritt 1) das Extrahieren von DNA aus der biologischen Probe und das Behandeln der extrahierten DNA mit einem Bisulfit umfasst, so dass unmethylierte Cytosin-Reste in der DNA desaminiert werden und methylierte Cytosin-Reste unverändert bleiben.

**3.** Verfahren nach Anspruch 1, wobei in Schritt 1) die Biomarker-Gene ALX4, BCAT1, BMP3, IKZF1, NDRG4, NPTX2, RARB, SDC2, Septin9 und VIM umfassen.

**4.** Verfahren nach Anspruch 1, wobei Schritt 1) das Nachweisen der Methylierungsgrade einer Zielregion innerhalb der Biomarker-Gene umfasst und wobei es sich bei der Zielregion um eine Nukleotidsequenz von mindestens 15 Basen in den Biomarker-Genen oder um eine komplementäre Sequenz davon handelt.

**5.** Verfahren nach Anspruch 1, wobei in Schritt 1)

der Nachweis des Methylierungsgrades des ALX4-Gens die Verwendung eines Primerpaares mit den in den SEQ ID NOs: 11 und 12 angegebenen Sequenzen oder eines Primerpaares mit den in den SEQ ID NOs: 15 und 16 angegebenen Sequenzen zur Durchführung einer PCR-Amplifikationsreaktion umfasst, wobei das ALX4-Gen oder ein Fragment davon, das in der biologischen Probe bisulfitbehandelt wurde, als Matrize dient;
der Nachweis des Methylierungsgrades des BCAT1-Gens die Verwendung eines Primerpaares mit den in den SEQ ID NOs: 19 und 20 angegebenen Sequenzen oder eines Primerpaares mit den in den SEQ ID NOs: 23 und 24 angegebenen Sequenzen zur Durchführung einer PCR-Amplifikationsreaktion umfasst, wobei das BCAT1-Gen oder ein Fragment davon, das in der biologischen Probe bisulfitbehandelt wurde, als Matrize dient;
der Nachweis des Methylierungsgrades des BMP3-Gens die Verwendung eines Primerpaars mit den in den SEQ ID NOs: 27 und 28 angegebenen Sequenzen oder eines Primerpaars mit den in den SEQ ID NOs: 31 und 32 angegebenen Sequenzen zur Durchführung einer PCR-Amplifikationsreaktion umfasst, wobei das BMP3-Gen oder ein Fragment davon, das in der biologischen Probe bisulfitbehandelt wurde, als Matrize dient;
der Nachweis des Methylierungsgrades des IKZF1-Gens die Verwendung eines Primerpaars mit den in den SEQ ID NOs: 35 und 36 angegebenen Sequenzen oder eines Primerpaars mit den in den SEQ ID NOs: 39 und 40 angegebenen Sequenzen zur Durchführung einer PCR-Amplifikationsreaktion umfasst, wobei das IKZF1-Gen oder ein Fragment davon, das in der biologischen Probe bisulfitbehandelt wurde, als Matrize dient;
der Nachweis des Methylierungsgrades des NDRG4-Gens die Verwendung eines Primerpaares mit den in den SEQ ID NOs: 43 und 44 angegebenen Sequenzen oder eines Primerpaares mit den in den SEQ ID NOs: 47 und 48 angegebenen Sequenzen zur Durchführung einer PCR-Amplifikationsreaktion umfasst, wobei das NDRG4-Gen oder ein Fragment davon, das in der biologischen Probe bisulfitbehandelt wurde, als Matrize dient;
der Nachweis des Methylierungsgrades des NPTX2-Gens die Verwendung eines Primerpaares mit den in SEQ

ID NOs: 51 und 52 angegebenen Sequenzen, eines Primerpaares mit den in SEQ ID NOs: 55 und 56 angegebenen Sequenzen oder eines Primerpaares mit den in SEQ ID NOs: 59 und 60 angegebenen Sequenzen zur Durchführung einer PCR-Amplifikationsreaktion umfasst, wobei das NPTX2-Gen oder ein Fragment davon, das in der biologischen Probe bisulfitbehandelt wurde, als Matrize dient;

der Nachweis des Methylierungsgrades des RARB-Gens die Verwendung eines Primerpaares mit den in den SEQ ID NOs: 63 und 64 angegebenen Sequenzen, eines Primerpaares mit den in den SEQ ID NOs: 67 und 68 angegebenen Sequenzen oder eines Primerpaares mit den in den SEQ ID NOs: 71 und 72 angegebenen Sequenzen zur Durchführung einer PCR-Amplifikationsreaktion umfasst, wobei das RARB-Gen oder ein Fragment davon, das in der biologischen Probe bisulfitbehandelt wurde, als Matrize dient;

der Nachweis des Methylierungsgrades des SDC2-Gens die Verwendung eines Primerpaares mit den in den SEQ ID NOs: 75 und 76 angegebenen Sequenzen, eines Primerpaares mit den in den SEQ ID NOs: 79 und 80 angegebenen Sequenzen oder eines Primerpaares mit den in den SEQ ID NOs: 83 und 84 angegebenen Sequenzen zur Durchführung einer PCR-Amplifikationsreaktion umfasst, wobei das SDC2-Gen oder ein Fragment davon, das in der biologischen Probe bisulfitbehandelt wurde, als Matrize dient;

der Nachweis des Methylierungsgrades des Septin9-Gens die Verwendung eines Primerpaares mit den in den SEQ ID NOs: 87 und 88 angegebenen Sequenzen oder eines Primerpaares mit den in den SEQ ID NOs: 91 und 92 angegebenen Sequenzen zur Durchführung einer PCR-Amplifikationsreaktion umfasst, wobei das Septin9-Gen oder ein Fragment davon, das in der biologischen Probe bisulfitbehandelt wurde, als Matrize dient; und

der Nachweis des Methylierungsgrades des VIM-Gens die Verwendung eines Primerpaares mit den in den SEQ ID NOs: 95 und 96 angegebenen Sequenzen oder eines Primerpaares mit den in den SEQ ID NOs: 99 und 100 angegebenen Sequenzen zur Durchführung einer PCR-Amplifikationsreaktion umfasst, wobei das VIM-Gen oder ein Fragment davon, das in der biologischen Probe bisulfitbehandelt wurde, als Matrize dient.

**6.** Verfahren nach Anspruch 5, wobei in Schritt 1)

der Nachweis des Methylierungsgrades des ALX4-Gens die Verwendung eines Primerpaares mit den in den SEQ ID NOs: 11 und 12 angegebenen Sequenzen und eines Blockierprimers mit der in SEQ ID NO: 13 angegebenen Sequenz oder eines Primerpaares mit den in den SEQ ID NOs: 15 und 16 angegebenen Sequenzen und eines Blockierprimers mit der in SEQ ID NO: 17 angegebenen Sequenz zur Durchführung einer PCR-Amplifikationsreaktion umfasst, wobei das bisulfitbehandelte ALX4-Gen oder ein Fragment davon in der biologischen Probe als Matrize dient;

der Nachweis des Methylierungsgrades des BCAT1-Gens die Verwendung eines Primerpaares mit den in SEQ ID NOs: 19 und 20 angegebenen Sequenzen und eines Blockierprimers mit der in SEQ ID NO: 21 angegebenen Sequenz oder eines Primerpaares mit den in SEQ ID NOs: 23 und 24 angegebenen Sequenzen und eines Blockierprimers mit der in SEQ ID NO: 25 angegebenen Sequenz zur Durchführung einer PCR-Amplifikationsreaktion umfasst, wobei das bisulfitbehandelte BCAT1-Gen oder ein Fragment davon in der biologischen Probe als Matrize dient;

der Nachweis des Methylierungsgrades des BMP3-Gens die Verwendung eines Primerpaares mit den in SEQ ID NOs: 27 und 28 angegebenen Sequenzen und eines Blockierprimers mit der in SEQ ID NO: 29 angegebenen Sequenz oder eines Primerpaares mit den in SEQ ID NOs: 31 und 32 angegebenen Sequenzen und eines Blockierprimers mit der in SEQ ID NO: 33 angegebenen Sequenz zur Durchführung einer PCR-Amplifikationsreaktion umfasst, wobei das bisulfitbehandelte BMP3-Gen oder ein Fragment davon in der biologischen Probe als Matrize dient;

der Nachweis des Methylierungsgrades des IKZF1-Gens die Verwendung eines Primerpaares mit den in SEQ ID NOs: 35 und 36 angegebenen Sequenzen und eines Blockierprimers mit der in SEQ ID NO: 37 angegebenen Sequenz oder eines Primerpaares mit den in SEQ ID NOs: 39 und 40 angegebenen Sequenzen und eines Blockierprimers mit der in SEQ ID NO: 41 angegebenen Sequenz zur Durchführung einer PCR-Amplifikationsreaktion umfasst, wobei das bisulfitbehandelte IKZF1-Gen oder ein Fragment davon in der biologischen Probe als Matrize dient;

der Nachweis des Methylierungsgrades des NDRG4-Gens die Verwendung eines Primerpaares mit den in den SEQ ID NOs: 43 und 44 angegebenen Sequenzen und eines Blockierprimers mit der in SEQ ID NO: 45 angegebenen Sequenz oder eines Primerpaares mit den in den SEQ ID NOs: 47 und 48 angegebenen Sequenzen und eines Blockierprimers mit der in SEQ ID NO: 49 angegebenen Sequenz zur Durchführung einer PCR-Amplifikationsreaktion umfasst, wobei das bisulfitbehandelte NDRG4-Gen oder ein Fragment davon in der biologischen Probe als Matrize dient;

der Nachweis des Methylierungsgrades des NPTX2-Gens die Verwendung eines Primerpaares mit den in den SEQ ID NOs: 51 und 52 angegebenen Sequenzen und eines Blockierprimers mit der in SEQ ID NO: 53 angegebenen Sequenz, eines Primerpaares mit den in den SEQ ID NOs: 55 und 56 angegebenen Sequenzen

und eines Blockierprimers mit der in SEQ ID NO: 57 angegebenen Sequenz, oder eines Primerpaares mit den in den SEQ ID NOs: 59 und 60 angegebenen Sequenzen und eines Blockierprimers mit der in SEQ ID NO: 61 angegebenen Sequenz zur Durchführung einer PCR-Amplifikationsreaktion umfasst, wobei das bisulfitbehandelte NPTX2-Gen oder ein Fragment davon in der biologischen Probe als Matrize dient;

der Nachweis des Methylierungsgrades des RARB-Gens die Verwendung eines Primerpaars mit den in den SEQ ID NOs: 63 und 64 angegebenen Sequenzen und eines Blockierprimers mit der in SEQ ID NO: 65 angegebenen Sequenz, eines Primerpaars mit den in den SEQ ID NOs: 67 und 68 angegebenen Sequenzen und eines Blockierprimers mit der in SEQ ID NO: 69 angegebenen Sequenz, oder eines Primerpaares mit den in den SEQ ID NOs: 71 und 72 angegebenen Sequenzen und eines Blockierprimers mit der in SEQ ID NO: 73 angegebenen Sequenz zur Durchführung einer PCR-Amplifikationsreaktion umfasst, wobei das bisulfitbehandelte RARB-Gen oder ein Fragment davon in der biologischen Probe als Matrize dient;

der Nachweis des Methylierungsgrades des SDC2-Gens die Verwendung eines Primerpaares mit den in den SEQ ID NOs: 75 und 76 angegebenen Sequenzen und eines Blockierprimers mit der in SEQ ID NO: 77 angegebenen Sequenz, eines Primerpaares mit den in den SEQ ID NOs: 79 und 80 angegebenen Sequenzen und eines Blockierprimers mit der in SEQ ID NO: 81 angegebenen Sequenz, oder eines Primerpaares mit den in den SEQ ID NOs: 83 und 84 angegebenen Sequenzen und eines Blockierprimers mit der in SEQ ID NO: 85 angegebenen Sequenz zur Durchführung einer PCR-Amplifikationsreaktion umfasst, wobei das bisulfitbehandelte SDC2-Gen oder ein Fragment davon in der biologischen Probe als Matrize dient;

der Nachweis des Methylierungsgrades des Septin9-Gens die Verwendung eines Primerpaares mit den in den SEQ ID NOs: 87 und 88 angegebenen Sequenzen und eines Blockierprimers mit der in SEQ ID NO: 89 angegebenen Sequenz oder eines Primerpaares mit den in den SEQ ID NOs: 91 und 92 angegebenen Sequenzen und eines Blockierprimers mit der in SEQ ID NO: 93 zur Durchführung einer PCR-Amplifikationsreaktion umfasst, wobei das bisulfitbehandelte Septin9-Gen oder ein Fragment davon in der biologischen Probe als Matrize dient; und

der Nachweis des Methylierungsgrades des VIM-Gens die Verwendung eines Primerpaares mit den in SEQ ID NOs: 95 und 96 angegebenen Sequenzen und eines Blockierprimers mit der in SEQ ID NO: 97 angegebenen Sequenz oder eines Primerpaares mit den in SEQ ID NOs: 99 und 100 angegebenen Sequenzen und eines Blockierprimers mit der in SEQ ID NO: 101 angegebenen Sequenz zur Durchführung einer PCR-Amplifikationsreaktion umfasst, wobei das bisulfitbehandelte VIM-Gen oder ein Fragment davon in der biologischen Probe als Matrize dient,

wobei die Blockierprimer eine 3'-Endmodifikation aufweisen, welche die Verlängerung und Amplifikation durch eine DNA-Polymerase verhindert.

**7.** Verfahren nach Anspruch 1, wobei Schritt 2) das Bestimmen des Darmkrebsstatus bei der Testperson anhand der Methylierungsgrade der Biomarker-Gene basierend auf einer logistischen Regression umfasst.

**8.** Kit zur Bestimmung eines Darmkrebsstatus bei einer Testperson, umfassend Primerpaare zum Nachweis von Methylierungsgraden von Biomarker-Genen in einer biologischen Probe der Testperson, wobei die Primerpaare zur Durchführung einer PCR-Amplifikationsreaktion mit den als Matrizen dienenden, bisulfitbehandelten Biomarker-Genen oder Fragmenten davon verwendet werden; und die Biomarker-Gene ALX4, BCAT1, IKZF1, NPTX2 und VIM umfassen.

**9.** Kit nach Anspruch 8, wobei die Biomarker-Gene ALX4, BCAT1, BMP3, IKZF1, NDRG4, NPTX2, RARB, SDC2, Septin9 und VIM umfassen.

**10.** Kit nach Anspruch 8, wobei

das zum Nachweis des Methylierungsgrades von ALX4 verwendete Primerpaar die in den SEQ ID NOs: 11 und 12 angegebenen Sequenzen aufweist oder die in den SEQ ID NOs: 15 und 16 angegebenen Sequenzen aufweist;

das zum Nachweis des Methylierungsgrades von BCAT1 verwendete Primerpaar die in den SEQ ID NOs: 19 und 20 angegebenen Sequenzen aufweist oder die in den SEQ ID NOs: 23 und 24 angegebenen Sequenzen aufweist;

das zum Nachweis des Methylierungsgrades von BMP3 verwendete Primerpaar die in den SEQ ID NOs: 27 und 28 angegebenen Sequenzen aufweist oder die in den SEQ ID NOs: 31 und 32 angegebenen Sequenzen aufweist;

das zum Nachweis des Methylierungsgrades von IKZF1 verwendete Primerpaar die in den SEQ ID NOs: 35 und 36 angegebenen Sequenzen aufweist oder die in den SEQ ID NOs: 39 und 40 angegebenen Sequenzen

aufweist;
das zum Nachweis des Methylierungsgrades von NDRG4 verwendete Primerpaar die in den SEQ ID NOs: 43 und 44 angegebenen Sequenzen aufweist oder die in den SEQ ID NOs: 47 und 48 angegebenen Sequenzen aufweist;
das zum Nachweis des Methylierungsgrades von NPTX2 verwendete Primerpaar die in SEQ ID NOs: 51 und 52 angegebenen Sequenzen aufweist, die in SEQ ID NOs: 55 und 56 angegebenen Sequenzen aufweist oder die in SEQ ID NOs: 59 und 60 angegebenen Sequenzen aufweist;
das zum Nachweis des Methylierungsgrades von RARB verwendete Primerpaar die in den SEQ ID NOs: 63 und 64 angegebenen Sequenzen aufweist, die in den SEQ ID NOs: 67 und 68 angegebenen Sequenzen aufweist oder die in den SEQ ID NOs: 71 und 72 angegebenen Sequenzen aufweist;
das zum Nachweis des Methylierungsgrades von SDC2 verwendete Primerpaar die in den SEQ ID NOs: 75 und 76 angegebenen Sequenzen aufweist, die in den SEQ ID NOs: 79 und 80 angegebenen Sequenzen aufweist oder die in den SEQ ID NOs: 83 und 84 angegebenen Sequenzen aufweist;
das zum Nachweis des Methylierungsgrades von Septin9 verwendete Primerpaar die in den SEQ ID NOs: 87 und 88 angegebenen Sequenzen aufweist oder die in den SEQ ID NOs: 91 und 92 angegebenen Sequenzen aufweist ; und
das zum Nachweis des Methylierungsgrades von VIM verwendete Primerpaar die in den SEQ ID NOs: 95 und 96 angegebenen Sequenzen aufweist oder die in den SEQ ID NOs: 99 und 100 angegebenen Sequenzen aufweist.

**11.** Kit nach Anspruch 10, das ferner einen Blockierprimer umfasst, wobei

der Blockierprimer, der in Kombination mit dem Primerpaar mit den in SEQ ID NO: 11 und 12 angegebenen Sequenzen verwendet wird, die in SEQ ID NO: 13 angegebene Sequenz aufweist;
der Blockierprimer, der in Kombination mit dem Primerpaar mit den in SEQ ID NO: 15 und 16 angegebenen Sequenzen verwendet wird, die in SEQ ID NO: 17 angegebene Sequenz aufweist;
der Blockierprimer, der in Kombination mit dem Primerpaar mit den in SEQ ID NO: 19 und 20 angegebenen Sequenzen verwendet wird, die in SEQ ID NO: 21 angegebene Sequenz aufweist;
der Blockierprimer, der in Kombination mit dem Primerpaar mit den in SEQ ID NO: 23 und 24 angegebenen Sequenzen verwendet wird, die in SEQ ID NO: 25 angegebene Sequenz aufweist;
der Blockierprimer, der in Kombination mit dem Primerpaar mit den in SEQ ID NO: 27 und 28 angegebenen Sequenzen verwendet wird, die in SEQ ID NO: 29 angegebene Sequenz aufweist;
der Blockierprimer, der in Kombination mit dem Primerpaar mit den in SEQ ID NO: 31 und 32 angegebenen Sequenzen verwendet wird, die in SEQ ID NO: 33 angegebene Sequenz aufweist;
der Blockierprimer, der in Kombination mit dem Primerpaar mit den in SEQ ID NO: 35 und 36 angegebenen Sequenzen verwendet wird, die in SEQ ID NO: 37 angegebene Sequenz aufweist;
der Blockierprimer, der in Kombination mit dem Primerpaar mit den in SEQ ID NO: 39 und 40 angegebenen Sequenzen verwendet wird, die in SEQ ID NO: 41 angegebene Sequenz aufweist;
der Blockierprimer, der in Kombination mit dem Primerpaar mit den in SEQ ID NO: 43 und 44 angegebenen Sequenzen verwendet wird, die in SEQ ID NO: 45 angegebene Sequenz aufweist;
der Blockierprimer, der in Kombination mit dem Primerpaar mit den in SEQ ID NO: 47 und 48 angegebenen Sequenzen verwendet wird, die in SEQ ID NO: 49 angegebene Sequenz aufweist;
der Blockierprimer, der in Kombination mit dem Primerpaar mit den in SEQ ID NO: 51 und 52 angegebenen Sequenzen verwendet wird, die in SEQ ID NO: 53 angegebene Sequenz aufweist;
der Blockierprimer, der in Kombination mit dem Primerpaar mit den in SEQ ID NO: 55 und 56 angegebenen Sequenzen verwendet wird, die in SEQ ID NO: 57 angegebene Sequenz aufweist;
der Blockierprimer, der in Kombination mit dem Primerpaar mit den in SEQ ID NO: 59 und 60 angegebenen Sequenzen verwendet wird, die in SEQ ID NO: 61 angegebene Sequenz aufweist;
der Blockierprimer, der in Kombination mit dem Primerpaar mit den in SEQ ID NO: 63 und 64 angegebenen Sequenzen verwendet wird, die in SEQ ID NO: 65 angegebene Sequenz aufweist;
der Blockierprimer, der in Kombination mit dem Primerpaar mit den in SEQ ID NO: 67 und 68 angegebenen Sequenzen verwendet wird, die in SEQ ID NO: 69 angegebene Sequenz aufweist;
der Blockierprimer, der in Kombination mit dem Primerpaar mit den in SEQ ID NO: 71 und 72 angegebenen Sequenzen verwendet wird, die in SEQ ID NO: 73 angegebene Sequenz aufweist;
der Blockierprimer, der in Kombination mit dem Primerpaar mit den in SEQ ID NO: 75 und 76 angegebenen Sequenzen verwendet wird, die in SEQ ID NO: 77 angegebene Sequenz aufweist;
der Blockierprimer, der in Kombination mit dem Primerpaar mit den in SEQ ID NO: 79 und 80 angegebenen Sequenzen verwendet wird, die in SEQ ID NO: 81 angegebene Sequenz aufweist;
der Blockierprimer, der in Kombination mit dem Primerpaar mit den in SEQ ID NO: 83 und 84 angegebenen

Sequenzen verwendet wird, die in SEQ ID NO: 85 angegebene Sequenz aufweist;
der Blockierprimer, der in Kombination mit dem Primerpaar mit den in SEQ ID NO: 87 und 88 angegebenen Sequenzen verwendet wird, die in SEQ ID NO: 89 angegebene Sequenz aufweist;
der Blockierprimer, der in Kombination mit dem Primerpaar mit den in SEQ ID NO: 91 und 92 angegebenen Sequenzen verwendet wird, die in SEQ ID NO: 93 angegebene Sequenz aufweist;
der Blockierprimer, der in Kombination mit dem Primerpaar mit den in SEQ ID NO: 95 und 96 angegebenen Sequenzen verwendet wird, die in SEQ ID NO: 97 angegebene Sequenz aufweist; und
der Blockierprimer, der in Kombination mit dem Primerpaar mit den in SEQ ID NO: 99 und 100 angegebenen Sequenzen verwendet wird, die in SEQ ID NO: 101 angegebene Sequenz aufweist,
wobei die Blockierprimer eine 3'-Endmodifikation aufweisen, die die Verlängerung und Amplifikation einer DNA-Polymerase verhindert.

**12.** Kit nach Anspruch 11, das ferner eine Sonde umfasst, wobei

die Sonde, die in Kombination mit dem Primerpaar mit den in SEQ ID NO: 11 und 12 angegebenen Sequenzen verwendet wird, die in SEQ ID NO: 14 angegebene Sequenz aufweist;
die Sonde, die in Kombination mit dem Primerpaar mit den in SEQ ID NO: 15 und 16 angegebenen Sequenzen verwendet wird, die in SEQ ID NO: 18 angegebene Sequenz aufweist;
die Sonde, die in Kombination mit dem Primerpaar mit den in SEQ ID NO: 19 und 20 angegebenen Sequenzen verwendet wird, die in SEQ ID NO: 22 angegebene Sequenz aufweist;
die Sonde, die in Kombination mit dem Primerpaar mit den in SEQ ID NO: 23 und 24 angegebenen Sequenzen verwendet wird, die in SEQ ID NO: 26 angegebene Sequenz aufweist;
die Sonde, die in Kombination mit dem Primerpaar mit den in SEQ ID NO: 27 und 28 angegebenen Sequenzen verwendet wird, die in SEQ ID NO: 30 angegebene Sequenz aufweist;
die Sonde, die in Kombination mit dem Primerpaar mit den in SEQ ID NO: 31 und 32 angegebenen Sequenzen verwendet wird, die in SEQ ID NO: 34 angegebene Sequenz aufweist;
die Sonde, die in Kombination mit dem Primerpaar mit den in SEQ ID NO: 35 und 36 angegebenen Sequenzen verwendet wird, die in SEQ ID NO: 38 angegebene Sequenz aufweist;
die Sonde, die in Kombination mit dem Primerpaar mit den in SEQ ID NO: 39 und 40 angegebenen Sequenzen verwendet wird, die in SEQ ID NO: 42 angegebene Sequenz aufweist;
die Sonde, die in Kombination mit dem Primerpaar mit den in SEQ ID NO: 43 und 44 angegebenen Sequenzen verwendet wird, die in SEQ ID NO: 46 angegebene Sequenz aufweist;
die Sonde, die in Kombination mit dem Primerpaar mit den in SEQ ID NO: 47 und 48 angegebenen Sequenzen verwendet wird, die in SEQ ID NO: 50 angegebene Sequenz aufweist;
die Sonde, die in Kombination mit dem Primerpaar mit den in SEQ ID NO: 51 und 52 angegebenen Sequenzen verwendet wird, die in SEQ ID NO: 54 angegebene Sequenz aufweist;
die Sonde, die in Kombination mit dem Primerpaar mit den in SEQ ID NO: 55 und 56 angegebenen Sequenzen verwendet wird, die in SEQ ID NO: 58 angegebene Sequenz aufweist;
die Sonde, die in Kombination mit dem Primerpaar mit den in SEQ ID NO: 59 und 60 angegebenen Sequenzen verwendet wird, die in SEQ ID NO: 62 angegebene Sequenz aufweist;
die Sonde, die in Kombination mit dem Primerpaar mit den in SEQ ID NO: 63 und 64 angegebenen Sequenzen verwendet wird, die in SEQ ID NO: 66 angegebene Sequenz aufweist;
die Sonde, die in Kombination mit dem Primerpaar mit den in SEQ ID NO: 67 und 68 angegebenen Sequenzen verwendet wird, die in SEQ ID NO: 70 angegebene Sequenz aufweist;
die Sonde, die in Kombination mit dem Primerpaar mit den in SEQ ID NO: 71 und 72 angegebenen Sequenzen verwendet wird, die in SEQ ID NO: 74 angegebene Sequenz aufweist;
die Sonde, die in Kombination mit dem Primerpaar mit den in SEQ ID NO: 75 und 76 angegebenen Sequenzen verwendet wird, die in SEQ ID NO: 78 angegebene Sequenz aufweist;
die Sonde, die in Kombination mit dem Primerpaar mit den in SEQ ID NO: 79 und 80 angegebenen Sequenzen verwendet wird, die in SEQ ID NO: 82 angegebene Sequenz aufweist;
die Sonde, die in Kombination mit dem Primerpaar mit den in SEQ ID NO: 83 und 84 angegebenen Sequenzen verwendet wird, die in SEQ ID NO: 86 angegebene Sequenz aufweist;
die Sonde, die in Kombination mit dem Primerpaar mit den in SEQ ID NO: 87 und 88 angegebenen Sequenzen verwendet wird, die in SEQ ID NO: 90 angegebene Sequenz aufweist;
die Sonde, die in Kombination mit dem Primerpaar mit den in SEQ ID NO: 91 und 92 angegebenen Sequenzen verwendet wird, die in SEQ ID NO: 94 angegebene Sequenz aufweist;
die Sonde, die in Kombination mit dem Primerpaar mit den in SEQ ID NO: 95 und 96 angegebenen Sequenzen verwendet wird, die in SEQ ID NO: 98 angegebene Sequenz aufweist; und

die Sonde, die in Kombination mit dem Primerpaar mit den in SEQ ID NO: 99 und 100 angegebenen Sequenzen verwendet wird, die in SEQ ID NO: 102 angegebene Sequenz aufweist,
wobei die Sonden an einem Ende eine fluoreszierende Gruppe und an dem anderen Ende eine fluoreszenzlöschende Gruppe aufweisen.

**13.** Kit nach Anspruch 8, das ferner eine Anleitung umfasst, die beschreibt, wie das Kit zu verwenden ist und wie die Nachweisergebnisse mittels einer logistischen Regression auszuwerten sind.

## Revendications

**1.** Procédé d'identification d'un état du cancer colorectal chez un sujet, comprenant:

1) la détection des niveaux de méthylation de gènes biomarqueurs dans un échantillon biologique prélevé sur le sujet, lesdits gènes biomarqueurs comprenant ALX4, BCAT1, IKZF1, NPTX2 et VIM; et
2) la comparaison des niveaux de méthylation détectés à l'étape 1) avec les niveaux de méthylation normaux des gènes biomarqueurs correspondants au sein d'une population, afin de déterminer l'étetdu cancer colorectal chez le sujet.

**2.** Procédé selon la revendication 1, dans lequel l'étape 1) comprend l'extraction d'ADN à partir de l'échantillon biologique et le traitement de l'ADN extrait avec un bisulfite, de sorte que des résidus de cytosine non méthylés dans l'ADN soient désaminés, et que des résidus de cytosine méthylés restent inchangés.

**3.** Procédé selon la revendication 1, dans lequel, à l'étape 1), les gènes biomarqueurs comprennent ALX4, BCAT1, BMP3, IKZF1, NDRG4, NPTX2, RARB, SDC2, Septin9 et VIM.

**4.** Procédé selon la revendication 1, dans lequel l'étape 1) comprend la détection des niveaux de méthylation d'une région cible au sein des gènes biomarqueurs, et dans lequel la région cible est une séquence nucléotidique d'au moins 15 bases dans les gènes biomarqueurs, ou une séquence complémentaire de celle-ci.

**5.** Procédé selon la revendication 1, dans lequel, à l'étape 1),

la détection du niveau de méthylation du gène ALX4 comprend l'utilisation d'une paire de primaires ayant les séquences décrites dans les SEQ ID NOs: 11 et 12 ou d'une paire de primaires ayant les séquences décrites dans les SEQ ID NOs: 15 et 16 pour réaliser une réaction d'amplification par PCR, en utilisant comme matrice le gène ALX4 ou un fragment de celui-ci qui est traité au bisulfite dans l'échantillon biologique;
la détection du niveau de méthylation du gène BCAT1 comprend l'utilisation d'une paire de primaires ayant les séquences décrites dans les SEQ ID NOs: 19 et 20 ou d'une paire de primaires ayant les séquences décrites dans les SEQ ID NOs: 23 et 24 pour réaliser une réaction d'amplification par PCR, en utilisant comme matrice le gène BCAT1 ou un fragment de celui-ci qui est traité au bisulfite dans l'échantillon biologique;
la détection du niveau de méthylation du gène BMP3 comprend l'utilisation d'une paire de primaires ayant les séquences décrites dans les SEQ ID NOs: 27 et 28 ou d'une paire de primaires ayant les séquences décrites dans les SEQ ID NOs: 31 et 32 pour réaliser une réaction d'amplification par PCR, en utilisant comme matrice le gène BMP3 ou un fragment de celui-ci qui est traité au bisulfite dans l'échantillon biologique;
la détection du niveau de méthylation du gène IKZF1 comprend l'utilisation d'une paire de primaires ayant les séquences décrites dans les SEQ ID NOs: 35 et 36 ou d'une paire de primaires ayant les séquences décrites dans les SEQ ID NOs: 39 et 40 pour réaliser une réaction d'amplification par PCR, en utilisant comme matrice le gène IKZF1 ou un fragment de celui-ci qui est traité au bisulfite dans l'échantillon biologique;
la détection du niveau de méthylation du gène NDRG4 comprend l'utilisation d'une paire de primaires ayant les séquences décrites dans les SEQ ID NOs: 43 et 44 ou d'une paire de primaires ayant les séquences d' s décrites dans les SEQ ID NOs: 47 et 48 pour réaliser une réaction d'amplification par PCR, en utilisant comme matrice le gène NDRG4 ou un fragment de celui-ci qui est traité a au bisulfite dans l'échantillon biologique;
la détection du niveau de méthylation du gène NPTX2 comprend l'utilisation d'une paire de primaires ayant les séquences décrites dans les SEQ ID NOs: 51 et 52, d'une paire de primaires ayant les séquences décrites dans les SEQ ID NOs: 55 et 56 ou d'une paire de primaires ayant les séquences décrites dans les SEQ ID NOs: 59 et 60 pour réaliser une réaction d'amplification par PCR, en utilisant comme matrice le gène NPTX2 ou un fragment de celui-ci qui est traité au bisulfite dans l'échantillon biologique;
la détection du niveau de méthylation du gène RARB comprend l'utilisation d'une paire de primaires ayant les

séquences décrites dans les SEQ ID NOs: 63 et 64, d'une paire de primaires ayant les séquences décrites dans les SEQ ID NOs: 67 et 68 ou d'une paire de primaires ayant les séquences décrites dans les SEQ ID N°: 71 et 72 pour réaliser une réaction d'amplification par PCR, en utilisant comme matrice le gène RARB ou un fragment de celui-ci qui est traité au bisulfite dans l'échantillon biologique;

la détection du niveau de méthylation du gène SDC2 comprend l'utilisation d'une paire de primaires ayant les séquences décrites dans les SEQ ID NOs: 75 et 76, d'une paire de primaires ayant les séquences décrites dans les SEQ ID NOs: 79 et 80 ou d'une paire de primaires ayant les séquences décrites dans les SEQ ID NOs: 83 et 84 pour réaliser une réaction d'amplification par PCR, en utilisant comme matrice le gène SDC2 ou un fragment de celui-ci qui est traité au bisulfite dans l'échantillon biologique;

la détection du niveau de méthylation du gène Septin9 comprend l'utilisation d'une paire de primaires ayant les séquences décrites dans les SEQ ID NOs: 87 et 88 ou d'une paire de primaires ayant les séquences décrites dans les SEQ ID NOs: 91 et 92 pour réaliser une réaction d'amplification par PCR, en utilisant comme matrice le gène Septin9 ou un fragment de celui-ci qui est traité au bisulfite dans l'échantillon biologique; et

la détection du niveau de méthylation du gène VIM comprend l'utilisation d'une paire de primaires ayant les séquences décrites dans les SEQ ID NOs: 95 et 96 ou d'une paire de primaires ayant les séquences décrites dans les SEQ ID NOs: 99 et 100 pour réaliser une réaction d'amplification par PCR, en utilisant comme matrice le gène VIM ou un fragment de celui-ci qui est traité au bisulfite dans l'échantillon biologique.

**6.** Procédé selon la revendication 5, dans lequel, à l'étape 1),

la détection du niveau de méthylation du gène ALX4 comprend l'utilisation d'une paire de primaires ayant les séquences décrites dans les SEQ ID NOs: 11 et 12 et d'un primaire de blocage ayant la séquence décrite dans la SEQ ID NO: 13, ou d'une paire de primaires ayant les séquences décrites dans les SEQ ID NOs: 15 et 16 et d'un primaires de blocage ayant la séquence décrite dans la SEQ ID NO: 17, pour réaliser une réaction d'amplification par PCR, en utilisant comme matrice le gène ALX4 traité au bisulfite ou un fragment de celui-ci présent dans l'échantillon biologique;

la détection du niveau de méthylation du gène BCAT1 comprend l'utilisation d'une paire de primaires ayant les séquences décrites dans les SEQ ID NO: 19 et 20 et d'un primaire de blocage ayant la séquence décrite dans la SEQ ID NO: 21, ou d'une paire de primaires ayant les séquences décrites dans les SEQ ID NO: 23 et 24 et d'un primaire de blocage ayant la séquence décrite dans la SEQ ID NO: 25, pour réaliser une réaction d'amplification par PCR, en utilisant comme matrice le gène BCAT1 traité au bisulfite ou un fragment de celui-ci présent dans l'échantillon biologique;

la détection du niveau de méthylation du gène BMP3 comprend l'utilisation d'une paire de primaires d' s ayant les séquences décrites dans les SEQ ID NO: 27 et 28 et d'un primaire de blocage ayant la séquence décrite dans la SEQ ID NO: 29, ou d'une paire de primaires ayant les séquences décrites dans les SEQ ID NO: 31 et 32 et d'un primaire de blocage ayant la séquence décrite dans la SEQ ID NO: 33, pour réaliser une réaction d'amplification par PCR, en utilisant comme matrice le gène BMP3 traité au bisulfite ou un fragment de celui-ci présent dans l'échantillon biologique;

la détection du niveau de méthylation du gène IKZF1 comprend l'utilisation d'une paire de primaires ayant les séquences décrites dans les SEQ ID NO :35 et 36 et d'un primaire de blocage ayant la séquence décrite dans la SEQ ID NO :37, ou d'une paire de primaires ayant les séquences décrites dans les SEQ ID NO: 39 et 40 et d'un primaire de blocage ayant la séquence décrite dans la SEQ ID NO: 41 pour réaliser une réaction d'amplification par PCR, en utilisant comme matrice le gène IKZF1 traité au bisulfite ou un fragment de celui-ci présent dans l'échantillon biologique;

la détection du niveau de méthylation du gène NDRG4 comprend l'utilisation d'une paire de primaires ayant les séquences décrites dans les SEQ ID NO: 43 et 44 et d'un primaire de blocage ayant la séquence décrite dans la SEQ ID NO: 45, ou d'une paire de primaires ayant les séquences décrites dans les SEQ ID NO: 47 et 48 et d'un primaire de blocage ayant la séquence décrite dans la SEQ ID NO: 49 pour réaliser une réaction d'amplification par PCR, en utilisant comme matrice le gène NDRG4 traité au bisulfite ou un fragment de celui-ci présent dans l'échantillon biologique;

la détection du niveau de méthylation du gène NPTX2 comprend l'utilisation d'une paire de primaires ayant les séquences décrites dans les SEQ ID NO: 51 et 52 et d'un primaire de blocage ayant la séquence décrite dans la SEQ ID NO: 53, d'une paire de primaires ayant les séquences décrites dans les SEQ ID NO: 55 et 56 et d'un primaire de blocage ayant la séquence décrite dans la SEQ ID NO: 57, ou d'une paire de primaires ayant les séquences décrites dans les SEQ ID NOs: 59 et 60 et d'un primaire de blocage ayant la séquence décrite dans la SEQ ID NO: 61, pour réaliser une réaction d'amplification par PCR, en utilisant comme matrice le gène NPTX2 traité au bisulfite ou un fragment de celui-ci présent dans l'échantillon biologique;

la détection du niveau de méthylation du gène RARB comprend l'utilisation d'une paire de primaires ayant les

séquences décrites dans les SEQ ID NOs: 63 et 64 et d'un primaire de blocage ayant la séquence décrite dans le SEQ ID NO: 65, d'une paire de primaires ayant les séquences décrites dans les SEQ ID NOs: 67 et 68 et d'un primaire de blocage ayant la séquence décrite dans le SEQ ID NO: 69, ou d'une paire de primaires ayant les séquences décrites dans les SEQ ID NOs: 71 et 72 et d'un primaire de blocage ayant la séquence décrite dans la SEQ ID NO: 73, pour réaliser une réaction d'amplification par PCR, en utilisant comme matrice le gène RARB traité au bisulfite ou un fragment de celui-ci présent dans l'échantillon biologique;

la détection du niveau de méthylation du gène SDC2 comprend l'utilisation d'une paire de primaires ayant les séquences décrites dans les SEQ ID NO: 75 et 76 et d'un primaire de blocage ayant la séquence décrite dans la SEQ ID NO: 77, d'une paire de primaires ayant les séquences décrites dans les SEQ ID NO: 79 et 80 et d'un primaire de blocage ayant la séquence décrite dans la SEQ ID NO: 81, ou d'une paire de primaires ayant les séquences décrites dans les SEQ ID NO: 83 et 84 et d'un primaire de blocage ayant la séquence décrite dans la SEQ ID NO: 85, pour réaliser une réaction d'amplification par PCR, en utilisant comme matrice le gène SDC2 traité au bisulfite ou un fragment de celui-ci présent dans l'échantillon biologique;

la détection du niveau de méthylation du gène Septin9 comprend l'utilisation d'une paire de primaires ayant les séquences décrites dans les SEQ ID NO: 87 et 88 et d'un primaire de blocage ayant la séquence décrite dans le SEQ ID NO: 89, ou d'une paire de primaires ayant les séquences décrites dans les SEQ ID NO: 91 et 92 et d'un primaire de blocage ayant la séquence décrite dans le SEQ ID NO: 93, pour réaliser une réaction d'amplification par PCR, en utilisant comme matrice le gène Septin9 traité au bisulfite ou un fragment de celui-ci présent dans l'échantillon biologique; et

la détection du niveau de méthylation du gène VIM comprend l'utilisation d'une paire de primaires ayant les séquences décrites dans les SEQ ID NO: 95 et 96 et d'un primaire de blocage ayant la séquence décrite dans la SEQ ID NO: 97, ou d'une paire de primaires ayant les séquences décrites dans les SEQ ID NO: 99 et 100 et d'un primaire de blocage ayant la séquence décrite dans la SEQ ID NO: 101, pour réaliser une réaction d'amplification par PCR, en utilisant comme matrice le gène VIM traité au bisulfite ou un fragment de celui-ci présent dans l'échantillon biologique,

les primaires de blocage présentant une modification à l'extrémité 3' qui empêche l'extension et l'amplification par une ADN polymérase.

**7.** Procédé selon la revendication 1, dans lequel l'étape 2) comprend la détermination de l'état de cancer colorectal chez le sujet en fonction des niveaux de méthylation des gènes biomarqueurs sur la base d'une régression logistique.

**8.** Kit destiné à identifier un état de cancer colorectal chez un sujet, comprenant des paires de primaires pour détecter les niveaux de méthylation de gènes biomarqueurs dans un échantillon biologique provenant du sujet, dans lequel les paires de primaires sont utilisées pour réaliser une réaction d'amplification par PCR avec, comme matrices, les gènes biomarqueurs ou des fragments de ceux-ci qui est traité au bisulfite; et les gènes biomarqueurs comprennent ALX4, BCAT1, IKZF1, NPTX2 et VIM.

**9.** Kit selon la revendication 8, dans lequel les gènes biomarqueurs comprennent ALX4, BCAT1, BMP3, IKZF1, NDRG4, NPTX2, RARB, SDC2, Septin9 et VIM.

**10.** Kit selon la revendication 8, dans lequel

la paire de primaires utilisée pour la détection du niveau de méthylation d'ALX4 présente les séquences décrites dans les SEQ ID NOs: 11 et 12 ou présente les séquences décrites dans les SEQ ID NOs: 15 et 16;

la paire de primaires utilisée pour la détection du niveau de méthylation de BCAT1 présente les séquences décrites dans les SEQ ID NOs: 19 et 20 ou présente les séquences décrites dans les SEQ ID NOs: 23 et 24;

la paire de primaires utilisée pour la détection du niveau de méthylation de BMP3 présente les séquences décrites dans les SEQ ID NOs :27 et 28 ou présente les séquences décrites dans les SEQ ID NOs :31 et 32;

la paire de primaires utilisée pour la détection du niveau de méthylation d' IKZF1 présente les séquences décrites dans les SEQ ID NOs :35 et 36 ou présente les séquences décrites dans les SEQ ID NOs: 39 et 40;

la paire de primaires utilisée pour la détection du niveau de méthylation de NDRG4 présente les séquences décrites dans les SEQ ID NOs: 43 et 44 ou présente les séquences décrites dans les SEQ ID NOs: 47 et 48;

la paire de primaires utilisée pour la détection du niveau de méthylation de NPTX2 présente les séquences décrites dans les SEQ ID NOs: 51 et 52, présente les séquences décrites dans les SEQ ID NOs: 55 et 56 ou présente les séquences décrites dans les SEQ ID NOs: 59 et 60;

la paire de primaires utilisée pour la détection du niveau de méthylation de RARB présente les séquences décrites dans les SEQ ID NOs: 63 et 64, les séquences décrites dans les SEQ ID NOs: 67 et 68, ou les séquences décrites dans les SEQ ID NOs: 71 et 72;

la paire de primaires utilisée pour la détection du niveau de méthylation de SDC2 présente les séquences décrites dans les SEQ ID NOs: 75 et 76, présente les séquences décrites dans les SEQ ID NOs: 79 et 80 ou présente les séquences décrites dans les SEQ ID NOs: 83 et 84;
la paire de primaires utilisée pour la détection du niveau de méthylation de Septin9 présente les séquences décrites dans les SEQ ID NOs: 87 et 88 ou présente les séquences décrites dans les SEQ ID NOs: 91 et 92 ; et
la paire de primaires utilisée pour la détection du niveau de méthylation de VIM présente les séquences décrites dans les SEQ ID NOs: 95 et 96 ou présente les séquences décrites dans les SEQ ID NOs: 99 et 100.

**11.** Kit selon la revendication 10, comprenant en outre un primaire de blocage, dans lequel

le primaire de blocage utilisée en combinaison avec la paire de primaires ayant les séquences décrites dans les SEQ ID NO: 11 et 12 présente la séquence décrite dans le SEQ ID NO: 13;
le primaire de blocage utilisée en combinaison avec la paire de primaires ayant les séquences décrites dans les SEQ ID NO: 15 et 16 présente la séquence décrite dans le SEQ ID NO: 17;
le primaire de blocage utilisée en combinaison avec la paire de primaires ayant les séquences décrites dans les SEQ ID NO: 19 et 20 présente la séquence décrite dans la SEQ ID NO: 21;
le primaire de blocage utilisée en combinaison avec la paire de primaires ayant les séquences décrites dans les SEQ ID NO: 23 et 24 présente la séquence décrite dans la SEQ ID N : 25;
le primaire de blocage utilisée en combinaison avec la paire de primaires ayant les séquences décrites dans les SEQ ID NO: 27 et 28 présente la séquence décrite dans la SEQ ID NO :29;
le primaire de blocage utilisée en combinaison avec la paire de primaires ayant les séquences décrites dans les SEQ ID NO: 31 et 32 présente la séquence décrite dans la SEQ ID NO: 33;
le primaire de blocage utilisée en combinaison avec la paire de primaires ayant les séquences décrites dans les SEQ ID NO: 35 et 36 présente la séquence décrite dans la SEQ ID NO: 37;
le primaire de blocage utilisée en combinaison avec la paire de primaires ayant les séquences décrites dans les SEQ ID NO: 39 et 40 présente la séquence décrite dans la SEQ ID NO: 41;
le primaire de blocage utilisée en combinaison avec la paire de primaires ayant les séquences décrites dans les SEQ ID NO: 43 et 44 présente la séquence décrite dans la SEQ ID NO: 45;
le primaire de blocage utilisée en combinaison avec la paire de primaires ayant les séquences décrites dans les SEQ ID NO: 47 et 48 présente la séquence décrite dans la SEQ ID NO: 49;
le primaire de blocage utilisée en association avec la paire de primaires ayant les séquences décrites dans les SEQ ID NO: 51 et 52 présente la séquence décrite dans la SEQ ID NO: 53;
le primaire de blocage utilisée en association avec la paire de primaires ayant les séquences décrites dans les SEQ ID NO: 55 et 56 présente la séquence décrite dans la SEQ ID NO: 57;
le primaire de blocage utilisée en association avec la paire de primaires ayant les séquences décrites dans les SEQ ID NO: 59 et 60 présente la séquence décrite dans la SEQ ID NO: 61;
le primaire de blocage utilisée en combinaison avec la paire de primaires ayant les séquences indiquées dans les SEQ ID NO: 63 et 64 présente la séquence décrite dans la SEQ ID NO: 6 ;
le primaire de blocage utilisée en combinaison avec la paire de primaires ayant les séquences décrite dans les SEQ ID NO: 67 et 68 présente la séquence décrite dans la SEQ ID NO: 69;
le primaire de blocage utilisée en combinaison avec la paire de primaires ayant les séquences décrite dans les SEQ ID NO: 71 et 72 présente la séquence décrite dans la SEQ ID NO : 73;
le primaire de blocage utilisée en combinaison avec la paire de primaires ayant les séquences décrites dans les SEQ ID NO: 75 et 76 présente la séquence décrite dans la SEQ ID NO: 77;
le primaire de blocage utilisée en combinaison avec la paire de primaires ayant les séquences décrites dans les SEQ ID NO: 79 et 80 présente la séquence décrite dans la SEQ ID NO: 81;
le primaire de blocage utilisée en combinaison avec la paire de primaires ayant les séquences décrites dans les SEQ ID NO: 83 et 84 présente la séquence décrite dans la SEQ ID NO: 85;
le primaire de blocage utilisée en combinaison avec la paire de primaires ayant les séquences décrites dans les SEQ ID NO: 87 et 88 présente la séquence décrite dans la SEQ ID NO: 89;
le primaire de blocage utilisée en combinaison avec la paire d'amorces ayant les séquences décrites dans les SEQ ID NO: 91 et 92 présente la séquence décrite dans la SEQ ID NO: 93;
le primaire de blocage utilisée en combinaison avec la paire de primaires ayant les séquences décrites dans les SEQ ID NO: 95 et 96 présente la séquence décrite dans la SEQ ID NO: 97; et
le primaire de blocage utilisée en combinaison avec la paire de primaires ayant les séquences décrites dans les SEQ ID NO: 99 et 100 présente la séquence décrite dans la SEQ ID NO: 101,
les primaires de blocage présentant une modification à l'extrémité 3' qui empêche l'extension et l'amplification par une ADN polymérase.

**12.** Kit selon la revendication 11, comprenant en outre une sonde, dans lequel

la sonde utilisée en combinaison avec la paire de primaires ayant les séquences décrites dans les SEQ ID NO: 11 et 12 présente la séquence décrite dans la SEQ ID NO: 14;
la sonde utilisée en combinaison avec la paire de primaires ayant les séquences décrites dans les SEQ ID NO: 15 et 16 présente la séquence décrite dans la SEQ ID NO: 18;
la sonde utilisée en combinaison avec la paire de primaires ayant les séquences décrites dans les SEQ ID NO: 19 et 20 présente la séquence décrite dans la SEQ ID NO: 22;
la sonde utilisée en association avec la paire de primaires ayant les séquences décrites dans les SEQ ID NO: 23 et 24 présente la séquence décrite dans la SEQ ID NO: 26;
la sonde utilisée en association avec la paire de primaires ayant les séquences décrites dans les SEQ ID NO: 27 et 28 présente la séquence décrite dans la SEQ ID NO: 30;
la sonde utilisée en association avec la paire de primaires ayant les séquences décrites dans les SEQ ID NO: 31 et 32 présente la séquence décrite dans la SEQ ID NO: 34;
la sonde utilisée en association avec la paire de primaires ayant les séquences décrites dans les SEQ ID NO: 35 et 36 présente la séquence décrite dans la SEQ ID NO: 38;
la sonde utilisée en association avec la paire de primaires dont les séquences sont décrites dans les SEQ ID NO: 39 et 40 présente la séquence décrite dans la SEQ ID NO: 42 ;
la sonde utilisée en association avec la paire de primaires ayant les séquences décrites dans les SEQ ID NO: 43 et 44 présente la séquence décrite dans la SEQ ID NO: 46;
la sonde utilisée en association avec la paire de primaires ayant les séquences décrites dans les SEQ ID NO: 47 et 48 présente la séquence décrite dans la SEQ ID NO: 50;
la sonde utilisée en association avec la paire de primaires ayant les séquences décrites dans les SEQ ID NO: 51 et 52 présente la séquence décrite dans la SEQ ID NO: 54;
la sonde utilisée en association avec la paire de primaires ayant les séquences décrites dans les SEQ ID NO: 55 et 56 présente la séquence décrite dans la SEQ ID NO: 58;
la sonde utilisée en association avec la paire de primaires ayant les séquences décrites dans les SEQ ID NO: 59 et 60 présente la séquence décrite dans la SEQ ID NO: 62;
la sonde utilisée en association avec la paire de primaires ayant les séquences décrites dans les SEQ ID NO: 63 et 64 présente la séquence décrite dans la SEQ ID NO: 66;
la sonde utilisée en association avec la paire de primaires ayant les séquences décrites dans les SEQ ID NO: 67 et 68 présente la séquence décrite dans la SEQ ID NO: 70;
la sonde utilisée en association avec la paire de primaires ayant les séquences décrites dans les SEQ ID NO: 71 et 72 présente la séquence décrite dans la SEQ ID NO: 74;
la sonde utilisée en association avec la paire de primaires ayant les séquences décrites dans les SEQ ID NO: 75 et 76 présente la séquence décrite dans la SEQ ID NO: 78;
la sonde utilisée en association avec la paire de primaires ayant les séquences décrites dans les SEQ ID NO: 79 et 80 présente la séquence décrite dans la SEQ ID NO: 82;
la sonde utilisée en association avec la paire de primaires ayant les séquences décrites dans les SEQ ID NO: 83 et 84 présente la séquence décrite dans la SEQ ID NO: 86;
la sonde utilisée en association avec la paire de primaires ayant les séquences décrites dans les SEQ ID NO: 87 et 88 présente la séquence décrite dans la SEQ ID NO: 90;
la sonde utilisée en association avec la paire de primaires ayant les séquences décrites dans les SEQ ID NO: 91 et 92 présente la séquence décrite dans la SEQ ID NO: 94;
la sonde utilisée en association avec la paire de primaires ayant les séquences décrites dans les SEQ ID NO: 95 et 96 présente la séquence décrite dans la SEQ ID NO: 98; et
la sonde utilisée en combinaison avec la paire de primaires ayant les séquences décrites dans les SEQ ID NO: 99 et 100 présente la séquence décrite dans la SEQ ID NO: 102,
les sondes comportant un groupe fluorescent à une extrémité et un groupe d'extinction de fluorescence à l'autre extrémité.

**13.** Kit selon la revendication 8, comprenant en outre une notice décrivant comment utiliser le kit et traiter les résultats de détection à l'aide d'une régression logistique.

ALX4
BCAT1
BMP3
IKZF1
NDRG4
NPTX2
RARB
SDC2
Septin9
VIM
sensitivity(%)
100-specificity(%)
0
20
40
60
80
100

Fig.1

(***) (***) (*) (**)
ALX4 (Ct)
50
40
30
20
no colorectal cancer
stage I
stage II
stage III
stage IV
colorectal cancer
(***) (***) (**) (ns)
BCAT1 (Ct)
(**) (**) (***) (**)
BMP3 (Ct)
(**) (**) (**) (***)
IKZF1 (Ct)
(*) (**) (**) (ns)
NDRG4 (Ct)
(**) (*) (*) (ns)
NPTX2 (Ct)

Fig.2A

(*) (ns) (**) (**)
RARB (Ct)
50
40
30
20
no colorectal cancer
stage I
stage II
stage III
stage IV
colorectal cancer
(**) (*) (**) (ns)
SDC2 (Ct)
(ns) (ns) (***) (*)
Septin9 (Ct)
(ns) (**) (*) (*)
VIM (Ct)


***: P value<0.001; **: 0.001<P value<0.01; *:0.01<P value<0.05; ns: P value>0.05.

Fig.2B

ALX4 (Ct)
BCAT1 (Ct)
BMP3 (Ct)
IKZF1 (Ct)
NDRG4 (Ct)
NPTX2 (Ct)
50
40
30
20
(***) (***) (**) (**)
(***) (**) (**) (*)
(***) (***) (***) (***)
(**) (**) (***) (ns)
(**) (**) (ns) (***)
(*) (ns) (**) (*)
no colorectal cancer
adenocarcinoma
mucoid carcinoma
undifferentiated carcinoma
other colorectal cancer
colorectal cancer


Fig.3A

RARB (Ct)
(**) (ns) (*) (**)
50
40
30
20
no colorectal cancer
adenocarcinoma
mucoid carcinoma
undifferentiated carcinoma
other colorectal cancer
colorectal cancer
SDC2 (Ct)
(**) (*) (**) (ns)
Septin9 (Ct)
(*) (*) (ns) (**)
VIM (Ct)
(*) (ns) (*) (*)

***: P value<0.001; **: 0.001<P value<0.01; *:0.01<P value<0.05; ns: P value>0.05.

Fig.3B

00
80
60
40
20
0
sensitivity(%)
0
20
40
60
80
100
100-specificity(%)

Fig.4

100
80
60
40
20
0
sensitivity(%)
0
20
40
60
80
100
100-specificity(%)

Fig.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011126768 A2
- RU 2630669 C1


### Non-patent literature cited in the description


- **RASMUSSEN SIMON L. et al.** Hypermethylated DNA, a circulating biomarker for colorectal cancer detection. *PLOS ONE*, 10 July 2017, vol. 12 (7), e0180809